(19) Europäisches Patentamt
European Patent Office
Office européen des brevets

(11) Veröffentlichungsnummer : **0 524 482 B1**

# (12) EUROPÄISCHE PATENTSCHRIFT

(45) Veröffentlichungstag der Patentschrift : **08.06.94 Patentblatt 94/23**

(51) Int. Cl.5 : **C07D 249/14,** A01N 43/653

(21) Anmeldenummer : **92111515.0**

(22) Anmeldetag : **07.07.92**

## (54) Substituierte Triazole und ihre Verwendung als Herbizide.

(30) Priorität : **20.07.91 DE 4124150**

(43) Veröffentlichungstag der Anmeldung : **27.01.93 Patentblatt 93/04**

(45) Bekanntmachung des Hinweises auf die Patenterteilung : **08.06.94 Patentblatt 94/23**

(84) Benannte Vertragsstaaten : **BE CH DE DK ES FR GB IT LI NL**

(56) Entgegenhaltungen : **EP-A- 0 332 991**

(73) Patentinhaber : **BAYER AG**
**D-51368 Leverkusen (DE)**

(72) Erfinder : **Findeisen, Kurt, Prof. Dir. Dr.**
**Dünfelder Strasse 28**
**W-5090 Leverkusen (DE)**
Erfinder : **Kuhnt, Dietmar, Dr.**
**Körnerstrasse 5**
**W-5090 Leverkusen (DE)**
Erfinder : **Müller, Klaus-Helmut, Dr.**
**Bockhackstrasse 55**
**W-4000 Düsseldorf 13 (DE)**
Erfinder : **König, Klaus, Dr.**
**Zum Hahnberg 40**
**W-5068 Odenthal (DE)**
Erfinder : **Santel, Hans-Joachim, Dr.**
**Grünstrasse 9a**
**W-5090 Leverkusen (DE)**
Erfinder : **Lürssen, Klaus, Dr.**
**August-Kierspel-Strasse 145**
**W-5060 Bergisch Gladbach 2 (DE)**
Erfinder : **Schmidt, Robert R., Dr.**
**Im Waldwinkel 110**
**W-5060 Bergisch Gladbach 2 (DE)**

**EP 0 524 482 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue substituierte Triazole, mehrere Verfahren zu ihrer Herstellung und ihre Verwendung als Herbizide.

Es ist bekannt, daß bestimmte substituierte Triazole wie beispielsweise die Verbindung 5-Dimethylamino-4-methyl-3-(4-phenylbut-2-ylaminocarbonyl)-1,2,4-triazol herbizide Eigenschaften besitzen (vergleiche z. B. DE 38 09 053).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue substituierte Triazole der allgemeinen Formel (I),

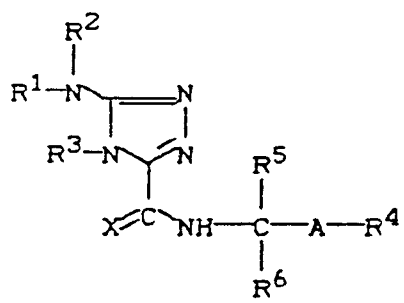

(I)

in welcher

| | |
|---|---|
| $R^1$ | für geradkettiges oder verzweigtes Alkyl mit 1 bis 6 Kohlenstoffatomen steht, |
| $R^2$ | für geradkettiges oder verzweigtes Alkyl mit 1 bis 6 Kohlenstoffatomen steht, |
| $R^3$ | für geradkettiges oder verzweigtes Alkyl mit 1 bis 6 Kohlenstoffatomen steht, |
| $R^4$ | für gegebenenfalls einfach oder mehrfach, gleich oder verschieden substituiertes Aryl mit 6 oder 10 Kohlenstoffatomen steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Dialkylamino mit jeweils 1 bis 4 Kohlenstoffatomen in den einzelnen geradkettigen oder verzweigten Alkylteilen, N-Alkanoylamino mit 1 bis 5 Kohlenstoffatomen im geradkettigen oder verzweigten Alkanoylteil, gegebenenfalls einfach oder mehrfach, gleich oder verschieden durch Halogen substituiertes, zweifach verknüpftes Dioxyalkylen mit 1 bis 3 Kohlenstoffatomen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl, Phenoxy, α-Naphthyl oder β-Naphthyl, und außerdem $R^4$ für Indanyl steht; |
| $R^5$ | entweder für Wasserstoff, für geradkettiges oder verzweigtes Alkyl mit 1 bis 6 Kohlenstoffatomen oder für Cyano steht und |
| $R^6$ | für Wasserstoff oder für geradkettiges oder verzweigtes Alkyl mit 1 bis 6 Kohlenstoffatomen steht oder |
| $R^5$ und $R^6$ | gemeinsam für zweifach verknüpftes Alkandiyl mit 2 bis 9 Kohlenstoffatomen stehen, |
| A | für einen Rest der Formel $-CH_2-CH_2-$; $-CH(CH_3)-CH_2-$; $-CH_2-O-$ steht und |
| X | für Sauerstoff oder Schwefel steht, |

wobei jedoch die Verbindung 5-Dimethylamino-4-methyl-3-(4-phenylbut-2-ylaminocarbonyl)-1,2,4-triazol ausgenommen ist,
gefunden.

Die Verbindungen der Formel (I) konnen gegebenenfalls in Abhangigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Triazole der allgemeinen Formel (I),

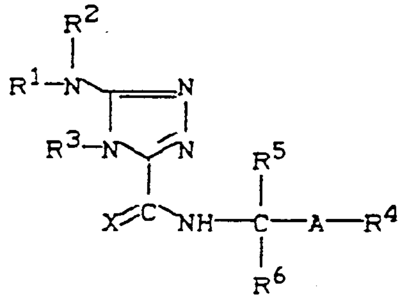

(I)

in welcher

| | |
|---|---|
| $R^1$ | für geradkettiges oder verzweigtes Alkyl mit 1 bis 6 Kohlenstoffatomen steht, |
| $R^2$ | für geradkettiges oder verzweigtes Alkyl mit 1 bis 6 Kohlenstoffatomen steht, |
| $R^3$ | für geradkettiges oder verzweigtes Alkyl mit 1 bis 6 Kohlenstoffatomen steht, |
| $R^4$ | für gegebenenfalls einfach oder mehr- fach, gleich oder verschieden substituiertes Aryl mit 6 oder 10 Kohlenstoffatomen steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Dialkylamino mit jeweils 1 bis 4 Kohlenstoffatomen in den einzelnen geradkettigen oder verzweigten Alkylteilen, N-Alkanoylamino mit 1 bis 5 Kohlenstoffatomen im geradkettigen oder verzweigten Alkanoylteil, gegebenenfalls einfach oder mehrfach, gleich oder verschieden durch Halogen substituiertes, zweifach verknüpftes Dioxyalkylen mit 1 bis 3 Kohlenstoffatomen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl, Phenoxy, α-Naphthyl oder β-Naphthyl, und außerdem $R^4$ für Indanyl steht; |
| $R^5$ | entweder für Wasserstoff, für geradkettiges oder verzweigtes Alkyl mit 1 bis 6 Kohlenstoffatomen oder für Cyano steht und |
| $R^6$ | für Wasserstoff oder für geradkettiges oder verzweigtes Alkyl mit 1 bis 6 Kohlenstoffatomen steht oder |
| $R^5$ und $R^6$ | gemeinsam für zweifach verknüpftes Alkandiyl mit 2 bis 9 Kohlenstoffatomen stehen, |
| A | für einen Rest der Formel $-CH_2-CH_2-$; $-CH(CH_3)-CH_2-$; $-CH_2-O-$ steht und |
| X | für Sauerstoff oder Schwefel steht, wobei jedoch die Verbindung 5-Dimethylamino-4-methyl-3-(4-phenylbut-2-ylaminocarbonyl)-1,2,4-triazol ausgenommen ist |

erhält, wenn man

a) Aminoguanidine der Formel (II),

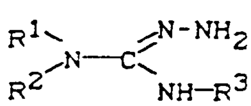

(II)

in welcher
$R^1$, $R^2$ und $R^3$ die oben angegebene Bedeutung haben,
oder deren Säureadditionssalze mit (Thio)Oxalesteramiden der Formel (III),

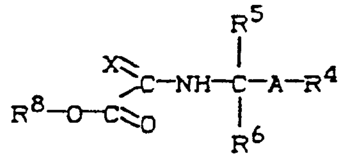

(III)

in welcher

$R^4$, $R^5$, $R^6$, X und A die oben angegebene Bedeutung haben und

$R^8$ für geradkettiges oder verzweigtes Alkyl mit 1 bis 4 Kohlenstoffatomen steht,

gegebenenfalls in Gegenwart eines Verdünnungsmittels und gegebenenfalls in Gegenwart eines Reaktionshilfsmittels umsetzt,

oder wenn man

b) substituierte Triazolyl(thio)carbonsäureester der Formel (IV),

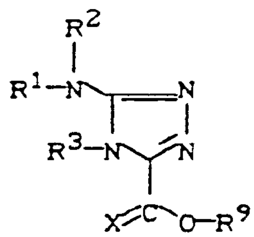

(IV)

in welcher

$R^1$, $R^2$, $R^3$ und X die oben angegebene Bedeutung haben und

$R^9$ für geradkettiges oder verzweigtes Alkyl mit 1 bis 4 Kohlenstoffatomen steht,

mit Aminen der Formel (V),

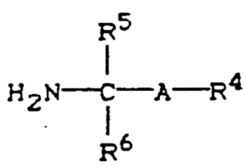

(V)

in welcher

$R^4$, $R^5$, $R^6$ und A die oben angegebene Bedeutung haben,

gegebenenfalls in Gegenwart eines Verdünnungsmittels umsetzt.

Schließlich wurde gefunden, daß die neuen substituierten Triazole der allgemeinen Formel (I) herbizide Eigenschaften besitzen.

Überraschenderweise zeigen die erfindungsgemäßen substituierten Triazole der allgemeinen Formel (I) eine erheblich bessere herbizide Wirksamkeit gegenüber Problemunkräutern und gleichzeitig eine vergleichbar gute Verträglichkeit gegenüber wichtigen Kulturpflanzen im Vergleich zu den aus dem Stand der Technik bekannten substituierten Triazolen, wie beispielsweise die Verbindung 5-Dimethylamino-4-methyl-3-(4-phenylbut-2-ylaminocarbonyl)-1,2,4-triazol, welche chemisch und wirkungsmäßig naheliegende Verbindungen sind.

Die erfindungsgemäßen substituierten Triazole sind durch die Formel (I) allgemein definiert. Bevorzugt sind Verbindungen der Formel (I), bei welchen

$R^1$ für geradkettiges oder verzweigtes Alkyl mit 1 bis 4 Kohlenstoffatomen steht,

$R^2$ für geradkettiges oder verzweigtes Alkyl mit 1 bis 4 Kohlenstoffatomen steht,

$R^3$ für geradkettiges oder verzweigtes Alkyl mit 1 bis 4 Kohlenstoffatomen steht,

$R^4$ für jeweils gegebenenfalls ein- bis dreifach, gleich oder verschieden substituiertes Phenyl, α-Naphthyl, β-Naphthyl oder Indanyl, steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Difluormethyl, Trifluormethyl, Difluormethoxy, Trifluormethoxy, Di-fluormethylthio, Trifluormethylthio, Dimethylamino, Diethylamino, N-Acetamido, Dioxymethylen, Difluordioxymethylen, Dioxyethylen, Trifluordioxyethylen, Tetrafluordioxyethylen, Methoxycarbonyl, Ethoxycarbonyl, Propoxycarbonyl, Methoximinomethyl, Methoximinoethyl, Ethoximinomethyl, Ethoximinoethyl oder jeweils gegebenenfalls ein- bis dreifach, gleich oder verschieden durch Fluor, Chlor, Brom, Methyl, Ethyl, Methoxy und/oder Ethoxy substituiertes Phenyl, Phenoxy, α-Naphthyl oder β-Naphthyl,

$R^5$ entweder für Wasserstoff, für geradkettiges oder verzweigtes Alkyl mit 1 bis 4 Kohlenstoffatomen oder für Cyano steht und

$R^6$ für Wasserstoff oder für geradkettiges oder verzweigtes Alkyl mit 1 bis 4 Kohlenstoffatomen steht oder

$R^5$ und $R^6$ gemeinsam für zweifach verknüpftes Alkandiyl mit 4 bis 9 Kohlenstoffatomen stehen,

A für einen der Reste $-CH_2-CH_2-$; $-CH(CH_3)-CH_2-$; $CH_2-O-$ steht und

X für Sauerstoff oder Schwefel steht,

wobei jedoch die Verbindung 5-Dimethylamino-4-methyl-3-(4-phenylbut-2-ylaminocarbonyl)-1,2,4-triazol ausgenommen ist.

Ganz besonders bevorzugt sind Verbindungen der Formel (I), bei welchen

$R^1$ für Methyl steht,

$R^2$ für Methyl oder Ethyl steht,

$R^3$ für Methyl oder Ethyl steht,

$R^4$ für gegebenenfalls ein- bis dreifach, gleich oder verschieden substituiertes Phenyl steht, wobei als Substituenten infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Difluormethyl, Trifluormethyl, Difluormethoxy, Trifluormethoxy, Di-fluormethylthio, Trifluormethylthio, Dimethylamino, Diethylamino, N-Acetamido, Dioxymethylen, Difluordioxymethylen, Dioxyethylen, Trifluordioxyethylen, Tetrafluordioxyethylen, Methoxycarbonyl, Ethoxycarbonyl, Propoxycarbonyl, Methoximinomethyl, Methoximinoethyl, Ethoximinomethyl, Ethoximinoethyl oder jeweils gegebenenfalls ein- bis dreifach, gleich oder verschieden durch Fluor, Chlor, Brom, Methyl, Ethyl, Methoxy und/oder Ethoxy substituiertes Phenyl, Phenoxy, α-Naphthyl oder β-Naphthyl,

$R^5$ für Wasserstoff, Methyl, Ethyl oder Cyano steht,

$R^6$ für Methyl, Ethyl, n- oder i-Propyl steht,

A für einen der Reste $-CH_2-CH_2-$; $-CH(CH_3)-CH_2-$; $-CH_2-O-$ steht und

X für Sauerstoff steht,

wobei jedoch die Verbindung 5-Dimethylamino-4-methyl-3-(4-phenylbut-2-ylaminocarbonyl)-1,2,4-triazol ausgenommen ist.

Im einzelnen sei auf die bei den Herstellungsbeispielen aufgeführten Verbindungen verwiesen.

Verwendet man beispielsweise 1-Amino-2,2,3-trimethylguanidinium-Hydrochlorid und Oxalsäuremonoethylester-[1-(2-chlorphenyl)-but-3-ylamid] als Ausgangsstoffe, so läßt sich der Reaktionsablauf des erfindungsgemäßen Verfahrens (a) durch das folgende Formelschema darstellen:

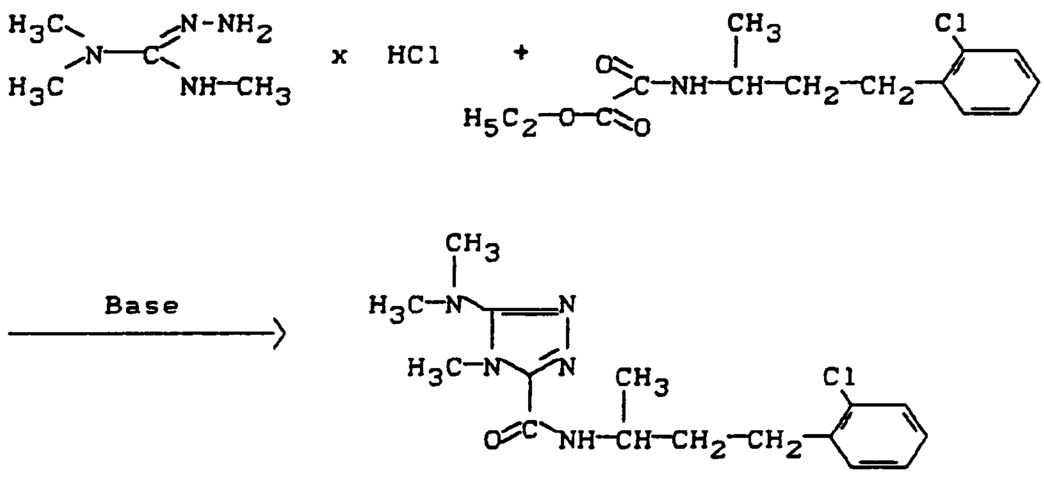

Verwendet man beispielsweise 5-Dimethylamino-3-methyl-1,2,4-triazol-3-yl-carbonsäuremethylester und 4-(4-Methylphenyl)-but-2-ylamin als Ausgangsstoffe, so läßt sich der Reaktionsablauf des erfindungsgemäßen Verfahrens (b) durch das folgende Formelschema darstellen:

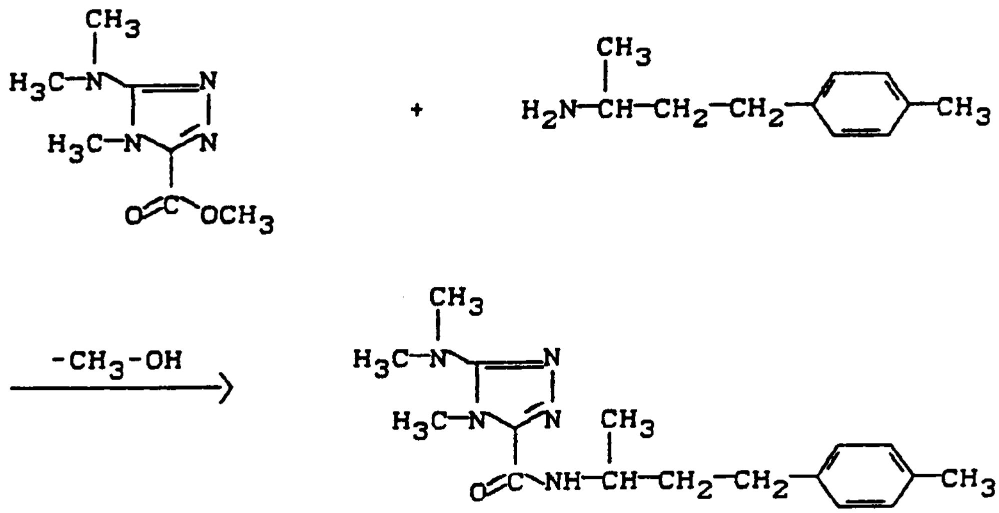


Die zur Durchführung des erfindungsgemäßen Verfahrens (a) als Ausgangsstoffe benötigten Aminoguanidine sind durch die Formel (II) allgemein definiert. In dieser Formel (II) stehen $R^1$, $R^2$ und $R^3$ vorzugsweise für diejenigen Reste, die bereits im Zusammenhang mit der Beschreibung der erfindungsgemäßen Verbindungen der Formel (I) als bevorzugt für diese Substituenten genannt wurden.
Die Aminoguanidine der Formel (II) und deren Säureadditionssalze, wie insbesondere deren Hydrochloride oder Hydrobromide sind bekannt (vergleiche z. B. J. Org. Chem, 19, 1807 [1954]; Bull. Soc, Chim Fr. 1975, 1649; US 2.845.458; DE 38 09 053).

Die zur Durchführung des erfindungsgemäßen Verfahrens (a) weiterhin als Ausgangsstoffe benötigten (Thio)Oxalesteramide sind durch die Formel (III) allgemein definiert. In dieser Formel (III) stehen $R^4$, $R^5$, $R^6$, X und A vorzugsweise für diejenigen Reste, die bereits im Zusammenhang sammenhang mit der Beschreibung der erfindungsgemäßen Verbindungen der Formel (I) als bevorzugt für diese Substituenten genannt wurden. $R^8$ steht vorzugsweise für Methyl oder Ethyl.

Die (Thio)Oxalerteramide der Formel (III) sind bekannt oder erhältlich in Analogie zu bekannten Verfahren (vergleiche z. B. EP 273 328; Ind. J. Chem. Sect. B, 24B, 940-947 [1985]; Acta Pharm. Suec., 20, 349-364 [1983] bzw. CA 100:174345; An. Quim. 73, 1177-1183 [1977] bzw. CA 89:129148; Bull. Soc. Chim. Belg. 85, 421-425 [1976]; Tetrahedron Lett. 1976, 2289-2290; Bull. Chem.Soc. Jpn. 60, 609-612 [1987] oder DE 38 09 053).

Die zur Durchführung des erfindungsgemäßen Verfahren (b) als Edukte erforderlichen substituierten Triazolyl-(thio)carbonsäureester sind durch die Formel (IV) allgemein definiert. In dieser Formel (IV) stehen $R^1$, $R^2$, $R^3$ und X vorzugsweise für diejenigen Reste, die bereits im Zusammenhang mit der Beschreibung der erfindungsgemäßen Stoffe der Formel (I) als bevorzugt für diese Substituenten genannt wurden. $R^9$ steht vorzugsweise für Methyl oder Ethyl.
Die substituierten (Thio)Oxalesteramide der Formel (IV) sind bekannt oder erhältlich in Analogie zu bekannten Verfahren (vergleiche z. B. Houben-Weyl, "Methoden der organischen Chemie", Band VIII, Seite 659, Thieme Verlag Stuttgart; US 2,857,390; Compt. Rend. Acad. Sci. 230, 848, [1950]; GB 15 78 719; DE 28 19 878; DE 12 27 451; DE 38 09 052).

Die zur Durchführung des erfindungsgemäßen Verfahrens (b) weiterhin als Edukte erforderlichen Amine sind durch die Formel (V) allgemein definiert. In dieser Formel (V) stehen $R^4$, $R^5$, $R^6$ und A vorzugsweise für diejenigen Reste, die bereits im Zusammenhang mit der Beschreibung der erfindungsgemäßen Stoffe der Formel (I) als bevorzugt für diese Substituenten genannt wurden. Die Amine der Formel (V) sind bekannt oder erhältlich in Analogie zu bekannten Verfahren (vergleiche z. B. EP 273 328; Ind. J Chem. Sect. B, 24B, 940-947 [1985]; Acta Pharm, Suec.,20, 349-364 [1983] bzw. CA 100:174345; An. Quim. 73, 1177-1183 [1977] bzw. CA 89:129148; Bull. Soc. Chim. Belg. 85, 421-425 [1976]; Tetrahedron Lett. 1976, 2289-2290; Bull. Chem. Soc. Jpn. 60, 609-612 [1987]).

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether wie Diethylether, Diisopropylether, Dioxan, Tetrahydrofuran oder Ethylenglykoldimethyl- oder -diethylether; Nitrile wie Acetonitril, Propionitril oder Benzonitril; Amide wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester oder Alkohole, wie Methanol, Ethanol, n- oder i-Propanol, n-, i-, s- oder t-Butanol, Ethylenglykolmonomethylether oder Ethylenglykolmonoethylether.

Das erfindungsgemäße Verfahren (a) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen +30°C und +150° C, vorzugsweise bei Temperaturen zwischen +50° C und +80° C.

Zur Durchführung des erfindungsgemäßen Verfahrens (a) setzt man pro Mol Aminoguanidin der Formel (II) bzw. eines entsprechenden Säureadditionssalzes im allgemeinen 1,0 bis 1,5 Mol, vorzugsweise 1,0 bis 1,2 Mol substituiertes (Thio)Oxalesteramid der Formel (III) und gegebenenfalls 1,0 bis 5,0 Mol, vorzugsweise 1,0 bis 2,5 Mol Base als Reaktionshilfsmittel ein. Die Reaktionsdurchführung, Aufarbeitung und Isolierung der Reaktionsprodukte erfolgt nach bekannten Verfahren (vergleiche hierzu beispielsweise DE 38 09 053 oder die Herstellungsbeispiele).

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl- oder -diethylether; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester.

Die Reaktionstemperaturen können bei der Durchführung des erfindungsgemäßen Verfahrens (b) in einem größeren Bereich variiert werden. Im allgemeinen arbeitet man bei Temperaturen zwischen +50° C und +250° C, vorzugsweise bei Temperaturen zwischen +100° C und +220° C.

Das erfindungsgemäße Verfahren (b) wird üblicherweise unter Normaldruck durchgeführt. Es ist jedoch auch möglich, unter erhöhtem Durck zu arbeiten; in diesem Fall arbeitet man vorzugsweise in Druckbereichen zwischen 1,0 und 50,0 bar, insbesondere in Druckbereichen zwischen 3,0 und 20,0 bar.

Zur Durchführung des erfindungsgemäßen Verfahrens (b) setzt man pro Mol substituiertem Triazolyl(thio)carbonsäureester der Formel (IV) im allgemeinen 1,0 bis 1,5 Mol, vorzugsweise 1,0 bis 1,2 Mol Amin der Formel (V) ein.

Die Reaktionsdurchführung, Aufarbeitung und Isolierung der Reaktionsprodukte erfolgt nach bekannten Verfahren (vergleiche hierzu beispielsweise DE 38 09 053 oder die Herstellungsbeispiele).

Die Reinigung der Endprodukte der Formel (I) erfolgt mit Hilfe üblicher Verfahren, beispielsweise durch Säulenchromatographie oder durch Umkristallisieren. Die Charakterisierung erfolgt mit Hilfe des Schmelzpunktes oder bei nicht kristallisierenden Verbindungen mit Hilfe der Protonen-Kernresonanzspektroskopie ($^1$H-NMR).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

<u>Dikotyle Kulturen der Gattungen:</u> Gossypium, Glycine, Beta, Daucus, Phaseolus, Pisum, Solanum, Linum,

Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von dikotylen Unkräutern in monokotylen Kulturen wie beispielsweise Weizen oder Mais einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden, Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Auch Mischungen mit 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitro-benzoesäure (ACIFLUORFEN); Chloressigsäure-N-(methoxymethyl)-2,6-diethylanilid (ALACHLOR); Methyl-6,6-dimethyl-2,4-dioxo-3-[1-(2-propenyloxyamino)-butyliden]-cyclohexancarbonsäure (ALLOXYDIM); 4-Amino-benzolsulfonyl-methylcarbamat (ASULAM); 2-Chlor-4-ethylamino-6-isopropylamino-1,3,5-triazin (ATRAZIN); 2-[[[[(4,6-Dimethoxypyrimidin-2-yl)-amino]-carbonyl]-amino)-sulfonyl]-methyl]- benzoesäuremethylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid (BUTACHLOR); N-(Butoxymethyl)-2-chlor-N-(2,6-diethylphenyl)-acetamid (BUTACHLOR); 5-Amino-4-chlor-2-phenyl-2,3-dihydro-3-oxy-pyridazin (CHLORIDAZON); Ethyl-2-{[(4-chlor-6-methoxy-2-pyrimidinyl)-aminocarbonyl)-aminosulfonyl}-benzoat (CHLORIMURON); N-(3-Chlorphenyl)-isopropylcarbamat (CHLORPROPHAM); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)- heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); N,S-Diethyl-N-cyclohexyl-thiolcarbamat (CYCLOATE); 2-[1-(Ethoximino)butyl]-3-hydroxy-5-[tetrahydro-(2H)-thiopyran-3-yl]-2-cyclohexen-1-on (CYCLOXYDIM); 2,6-Dichlorbenzonitril (DICHLOBENIL); 2-[4-(2,4-Dichlorphenoxy)-phenoxy)-propionsäure, deren Methyl- oder deren Ethylester (DICLOFOP); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl-oder deren Ethylester (FENOXAPROP); 2-[4-(5-Trifluormethyl-2-pyridyloxy)-phenoxy)-propansäure oder deren Butylester (FLUAZIFOP); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); [(4-Amino-3,5-dichlor-6-fluor-2-pyridinyl)-oxy)-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); N-Phosphonomethyl-glycin (GLYPHOSATE); 2-{4-[(3-Chlor-5-(trifluormethyl)-2-pyridinyl)-oxy)-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 2-(4,5-Dihydro-4-methyl-4-isopropyl-5-oxo-1H-imidazol-2-yl)-pyridin-3-carbonsäure (IMAZAPYR); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-N'-(4-isopropylphenyl)-harnstoff (ISOPROTURON); (2-Ethoxy-1-methyl-2-oxo-ethyl)-5-[2-chlor-4-(trifluormethyl)-phenoxy]-2-nitro benzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[(( 4-Methoxy-6-methyl-1,3,5-triazin-2-yl)-amino)-carbonyl)-amino]-sulfonyl}-benzoesäure oder deren Methylester (METSULFURON); S-Ethyl-N,N-hexamethylen-thiolcarbamat (MOLINATE); 1-(3-Trifluormethyl-phenyl)-4-methylamino-5-chlor-6-pyridazon (NORFLURAZON); 4-(Di-n-propylamino)-3,5-dinitrobenzolsulfonamid (ORYZALIN); (2-Chlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); 3-(Ethoxycarbonylaminophenyl)-N-(3'-methylphenyl)-carbamat (PHENMEDIPHAM); 4-Amino-3,5,6-trichlorpyridin-2-carbonsäure (PICLORAM); α-Chlor-2',6'-diethyl-N-(2-propoxyethyl)-acetanilid (PRETILACHLOR); 2-Chlor-N-isopropylacetanilid (PROPACHLOR); Isopropyl-N-phenyl-carbamat (PROPHAM); 0-(6-Chlor-3-phenyl-pyridazin-4-yl)-S-octyl-thiocarbonat (PYRIDATE); 2-[4-(6-Chlorchinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); S-[(4-Chlorphenyl)methyl]-N,N-diethyl-thiocarbamat (THIOBENCARB); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN); N-(2,4-Difluorphenyl-2-[3-trifluormethylphenoxy]-3-pyridincarboxamid (DIFLUFENICAN); N-(3,4-Dichlorphenyl)-propionanilid (PROPANIL); 3,5,6-Trichlor-2-pyridyloxyessigsäure (TRICLOPYR); 2-Methoxy-3,6-dichlorbenzoesäure bzw. deren Methylester (DICAMBA); 3-(Methoxycarbonylaminophenyl)-N-phenyl-carbamat (DESMEDIPHAM); 2-Ethyl-6'-methyl-N-ethoxymethyl-2-chloracetanilid (ACETOCHLOR); 2-Ethoxy-2-oxoethyl-5-(2-chlor-4-trifluormethylphenoxy)-2-nitrobenzoat (FLUOROGLYCOFEN); 5-(2-Chlor-6-fluor-4-trifluormethylphenoxy)-N-ethylsulfonyl-2-nitrobenzamid (HALOSAFEN); 2-[1-(Ethoximino)-propyl]-3-hydroxy-5-(2,4,6-trimethylphenyl)-2-cyclohexen-1-on (TRALKOXYDIM); N-[[[[(4,6-Dimethoxy-2-pyrimidinyl)-amino]-carbonyl]-amino]-sulfonyl]-N-methyl-methansulfonamid (AMIDOSULFURON); 2-(2-Methoxy-ethoxy-)-N-[[(4,6-dimethoxy-1,3,5-triazin-2-yl)-amino]-carbonyl]-benzolsulfonamid (CINOSULFURON); 2-[[[[(4,6-Dimethoxy-2-pyrimidinyl)-amino]-carbonyl]-amino]-sulfonyl]-N,N-dimethyl-3-pyridincarboxamid (NICOSULFURON); Ethyl-5-[[[[(4,6-dimethoxy-2-pyrimidinyl)-amino]-carbonyl]-amino]-sulfonyl)-1-methyl-1H-pyrazol-4- carboxylat (PYRAZOSULFURON); 3-[[[[(4-Methoxy-6-methyl-1,3,5-triazin-2-yl)-amino]-carbonyl]-amino]-sulfonyl]-thiophen-2-carbonsäure oder deren Methylester (THIAMETURON, THIFENSULFURON); 2-(2-Chlorethoxy-)-N-[[(4-methoxy-6-methyl-1,3,5-triazin-2-yl)-amino]-carbonyl]-benzolsulfonamid (TRIASULFURON); Methyl-2-[[[[(4-methoxy-6-methyl-1,3,5-triazin-2-yl)-methylamino]-carbonyl]-amino]-sulfonyl]-benzoat (TRIBENURON); S-(Phenylmethyl)-(1,2-dimethylpropyl)-ethyl-thiocarbamat (ESPROCARB); S-(Phenylmethyl)-dipropyl-thiocarbamat (PROSULFOCARB); 4-Ethylamino-2-t-butylamino-6-chlor-1,3,5-triazin (TERBUTYLAZIN); (2,3-Dihydro-3,3-dimethyl-5-benzofuranyl)-ethansulfonat (BENFURESATE); 2-[(2-Chlorphenyl)-methyl)-4,4-dimethyl-3-isoxazolidinon (CLOMAZONE, DIMETHAZONE); S,S-Dimethyl-2-difluormethyl-4-(2-methylpropyl)-6-trifluormethyl-3,5-pyridindicarbothioat (DITHIOPYR); N-[3-(1-Ethyl-1-methylpropyl)-isoxazol-5-yl]-2,6-dimethoxybenzamid (ISOXABEN); 3,7-Dichlor-8-chinolincarbonsäure (QUINCHLORAC); 7-Chlor-3-methyl-8-chinolincarbonsäure (QINMERAC) oder N-Phosphonomethylglycin Trimethylsulfoniumsalz (SULFOSATE) sind gegebenenfalls von Vorteil.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen; Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro Hektar.

Die Herstellung und die Verwendung der erfindungsgemäßen Wirkstoffe geht aus den nachfolgenden Beispielen hervor.

Herstellungsbeispiele:

Beispiel 1:

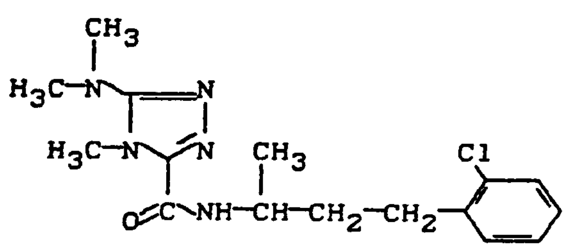

(Verfahren a)

7,6 g (0,05 Mol) 1-Amino-2,2,3-trimethylguanidinium Hydrochlorid, 14,15 g (0,05 Mol) Oxalsäuremonoethylester-1-(2-chlorphenyl)-3-butylamid und 5,4 g (0,1 Mol) Natriummethylat werden in 200 ml Ethanol 4 Stunden bei Rückflußtemperatur gerührt, anschließend auf Raumtemperatur abgekühlt, filtriert, das Filtrat im Vakuum eingeengt, der Rückstand in Dichlormethan aufgenommen, dreimal mit jeweils 50 ml Wasser gewaschen, über Natriumsulfat getrocknet und im Vakuum eingeengt. Der Rückstand wird über Kieselgel chromatographiert (Laufmittel: Cyclohexan/Essigester 1:1).

Man erhält 9,5 g (57 % der Theorie) 5-Dimethylamino-4-methyl-4H-1,2,4-triazol-3-ylcarbonsäure-1-(2-chlorphenyl)-3-butylamid als Öl.

$^1$H-NMR ($CDCl_3$/Tetramethylsilan):

$\delta$ = 1,82-1,90; 2,76-2,85; 2,90; 3,80 ppm.

Herstellung der Ausgangsverbindung:

Beispiel II-1:

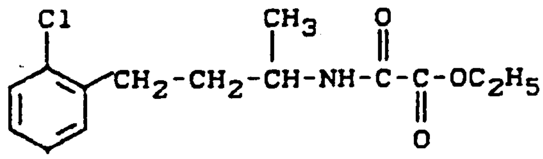

Zu 54,9 g (0,3 Mol) 1-(2-Chlorphenyl)-3-butylamin (vergleiche z. B. EP 6614) und 30,3 g (0,3 Mol) Triethylamin in 400 ml Methylenchlorid gibt man bei Raumtemperatur tropfenweise unter Rühren und Eiskühlung 40,8 g (0,3 Mol) Oxalsäureethylesterchlorid zu, rührt nach beendeter Zugabe 30 Minuten bei Raumtemperatur und filtriert anschließend ausgefallenes Triethylaminhydrochlorid ab. Das Filtrat wird dreimal mit jeweils 100 ml Wasser gewaschen, über Natriumsulfat getrocknet und im Vakuum eingeengt.

Man erhalt 82,2 g (97 % der Theorie) Oxalsäuremonoethylester-1-(2-chlorphenyl)-3-butylamid als Öl.

$^1H$-NMR ($CDCl_3$/Tetramethylsilan):

$\delta$ = 1,25-1,28; 1,8-1,88; 4,02-4,12; 4,3-4,4; 7,1-7,25 ppm.

Beispiel 2:

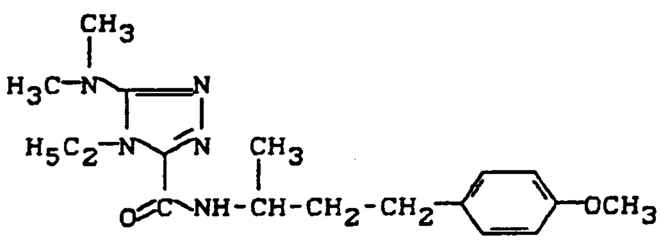

Verfahren (a)

8,33 g (0,05 Mol) 1-Amino-2,2-dimethyl-3-ethylguanidinium Hydrochlorid, 14,0 g (0,05 Mol) Oxalsäuremonoethylester-1-(4-methoxyphenyl)-3-butylamid und 5,4 g (0,1 Mol) Natriummethylat werden in 200 ml Ethanol 4 Stunden bei Rückflußtemperatur gerührt, anschließend auf Raumtemperatur abgekühlt, filtriert, das Filtrat im Vakuum eingeengt, der Rückstand in Dichlormethan aufgenommen, dreimal mit jeweils 50 ml Wasser gewaschen, über Natriumsulfat getrocknet und im Vakuum eingeengt. Der Rückstand wird über Kieselgel chromatographiert (Laufmittel: Cyclohexan / Essigester 1:1).

Man erhält 10,4 g (60 % der Theorie) 5-Dimethylamino-4-ethyl-4H-1,2,4-triazol-3-ylcarbonsäure-1-(4-methoxyphenyl)-3-butylamid vom Schmelzpunkt Fp. 39-40°C.

Herstellung der Ausgangsverbindung:

Beispiel II-2:

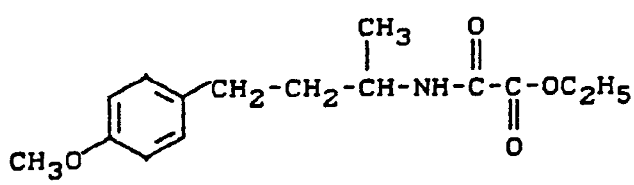

Zu 35,8 g (0,2 Mol) 1-(4-Methoxyphenyl)-3-butylamin (vergleiche z. B. EP 6614) und 20,2 g (0,3 Mol) Triethylamin in 300 ml Methylenchlorid gibt man bei Raumtemperatur tropfenweise unter Rühren und Eiskühlung 27,2 g (0,2 Mol) Oxalsäureethylesterchlorid zu, rührt nach beendeter Zugabe 30 Minuten bei Raumtemperatur und filtriert anschließend ausgefallenes Triethylaminhydrochlorid ab. Das Filtrat wird dreimal mit jeweils 100 ml Wasser gewaschen, über Natriumsulfat getrocknet und im Vakuum eingeengt.

Man erhält 54,3 g (97 % der Theorie) Oxalsäuremonoethylester-1-(4-methoxyphenyl)-3-butylamid als Öl.
$^1$H-NMR ($CDCl_3$/Tetramethylsilan):
δ = 1,21-1,23; 1,78-1,83; 3,88; 4,3-4,38; 7,05-7,10 ppm.

In entsprechender Weise und gemäß den allgemeinen Angaben zur Herstellung erhält man die folgenden substituierten Triazole der allgemeinen Formel (I):

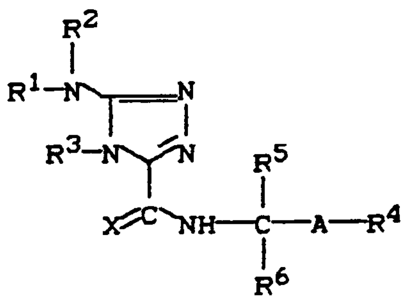

(I)

| Bsp.-Nr. | 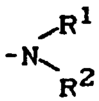 | $R^3$ | $R^4$ | $R^5$ | $R^6$ | A | X | physikalische Eigenschaften |
|---|---|---|---|---|---|---|---|---|
| 3 | 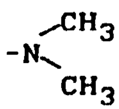 | $CH_3$ | 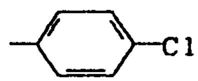 | H | $CH_3$ | 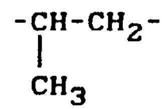 | O | $^1$H-NMR*): 1,22-1,25; 1,9-2,0; 2,9; 3,8 |
| 4 | 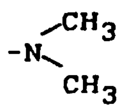 | $CH_3$ | 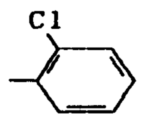 | H | $CH_3$ | $-CH_2-CH_2-$ | O | $^1$H-NMR*): 1,25-1,27; 2,3; 2,9; 3,8; 7,08; 7,32-7,36 |
| 5 | 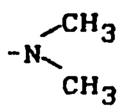 | $CH_3$ | 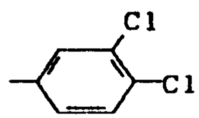 | H | $CH_3$ | $-CH_2-CH_2-$ | O | $^1$H-NMR*): 1,25-1,30; 2,6-2,7; 2,9; 3,8; 7,56-7,60 |
| 6 | 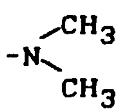 | $CH_3$ | 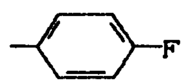 | H | $CH_3$ | $-CH_2-CH_2-$ | O | $^1$H-NMR*): 1,25-1,28; 2,6-2,7; 2,9-3,8; 4,1-4,2 |
| 7 | 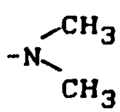 | $CH_3$ | 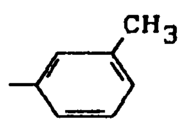 | H | $CH_3$ | 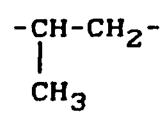 | O | $^1$H-NMR*): 0,85-0,9; 1,23-1,27; 2,3; 2,9; 3,8 |
| 8 | 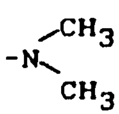 | $CH_3$ | 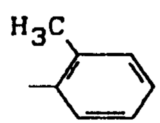 | H | $CH_3$ | 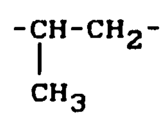 | O | $^1$H-NMR*): 1,25-1,28; 1,9-2,05; 2,9; 3,8; 7,1; 7,25 |

| Bsp.-Nr. | 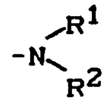 | $R^3$ | $R^4$ | $R^5$ | $R^6$ | A | X | physikalische Eigenschaften |
|---|---|---|---|---|---|---|---|---|
| 9 | 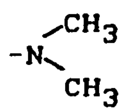 | $CH_3$ | 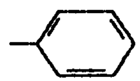 | H | $CH_3$ | 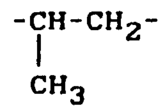 | O | $^1$H-NMR*): 0,85-0,9; 2,4-2,5; 2,9; 3,8 7,1-7,3 |
| 10 | 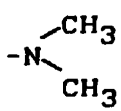 | $CH_3$ | 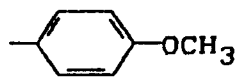 | H | $CH_3$ | $-CH_2-CH_2-$ | O | $^1$H-NMR*): 1,25-1,27; 2,3-2,4; 2,9; 3,75; 3,8 |
| 11 | 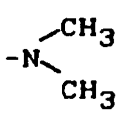 | $CH_3$ | 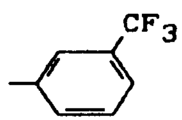 | H | $CH_3$ | 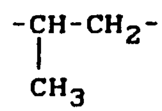 | O | $^1$H-NMR*): 0,85-0,9; 1,95-2,05; 2,9; 3,8; 7,3-7,45 |
| 12 | 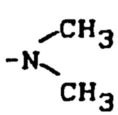 | $CH_3$ | 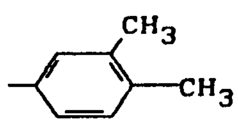 | H | $CH_3$ | 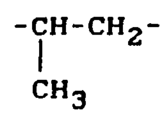 | O | $^1$H-NMR*): 0,85-0,9; 2,2; 2,9; 3,8; 7,8-7,85 |
| 13 | 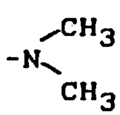 | $CH_3$ | 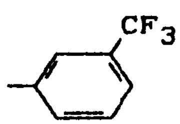 | H | $CH_3$ | $-CH_2-CH_2-$ | O | $^1$H-NMR*): 1,29-1,32; 1,85-1,95; 2,9; 3,8; 4,12-4,22 |
| 14 | 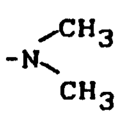 | $CH_3$ | 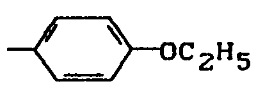 | H | $CH_3$ | $-CH_2-CH_2-$ | O | $^1$H-NMR*): 1,25-1,27; 1,38-1,42; 2,6-2,67; 2,9; 3,8 |

| Bsp.-Nr. | $-N\langle^{R^1}_{R^2}$ | $R^3$ | $R^4$ | $R^5$ | $R^6$ | A | X | physikalische Eigenschaften |
|---|---|---|---|---|---|---|---|---|
| 16 | $-N\langle^{CH_3}_{CH_3}$ | $CH_3$ | 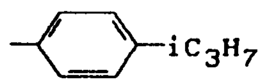 | H | H | $-CH-CH_2-$<br>$CH_3$ | O | $^1H$-NMR*): 0,95-0,97; 1,22-1,25; 2,4-2,5; 2,9; 3,8 |
| 17 | $-N\langle^{CH_3}_{CH_3}$ | $CH_3$ | 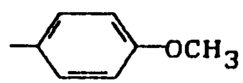 | H | $CH_3$ | $-CH_2-O-$ | O | $^1H$-NMR*): 1,4-1,43; 2,9; 3,75; 3,8; 3,95-4,03 |
| 18 | $-N\langle^{CH_3}_{CH_3}$ | $CH_3$ | 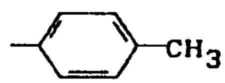 | H | $CH_3$ | $-CH_2-O-$ | O | $^1H$-NMR*): 1,4-1,43; 2,28; 2,9; 3,8; 3,95-4,05 |
| 19 | $-N\langle^{CH_3}_{CH_3}$ | $CH_3$ | 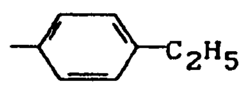 | H | $CH_3$ | $-CH_2-CH_2-$ | O | $^1H$-NMR*): 1,2-1,25; 1,26-1,29; 1,8-1,9; 2,9; 3,8 |
| 20 | $-N\langle^{CH_3}_{CH_3}$ | $C_2H_5$ | 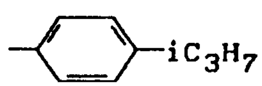 | H | H | $-CH-CH_2-$<br>$CH_3$ | O | $^1H$-NMR*): 0,95-0,98; 1,2-1,25; 1,4-1,45; 2,9; 4,25-4,32 |

| Bsp.-Nr. | 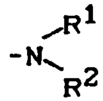 | $R^3$ | $R^4$ | $R^5$ | $R^6$ | A | X | physikalische Eigenschaften |
|---|---|---|---|---|---|---|---|---|
| 21 | 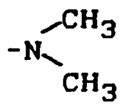 | $C_2H_5$ | 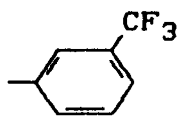 | H | $CH_3$ | 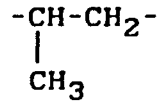 | O | $^{1}H$-NMR*): 1,25-1,28; 2,36-2,45; 2,9; 4,25-4,35 |
| 22 | 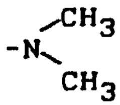 | $C_2H_5$ | 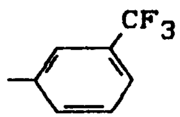 | H | $CH_3$ | $-CH_2-CH_2-$ | O | $^{1}H$-NMR*): 1,28-1,32; 1,4-1,48; 2,7-2,8; 2,9 |
| 23 | 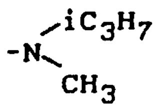 | $CH_3$ | 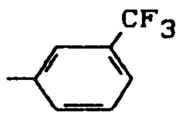 | H | $CH_3$ | $-CH_2-CH_2-$ | O | $^{1}H$-NMR*): 1,2-1,23; 1,26-1,3; 2,8; 3,75 |
| 24 | 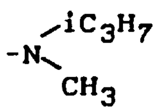 | $CH_3$ | 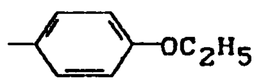 | H | $CH_3$ | $-CH_2-CH_2-$ | O | $^{1}H$-NMR*): 1,2-1,23; 1,38-1,43; 2,7; 3,75; 3,95-4,05 |
| 25 | 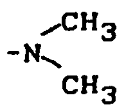 | $CH_3$ | 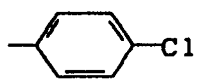 | H | H | 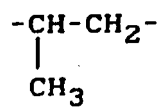 | O | $^{1}H$-NMR*): 0,9-0,93; 2,9; 3,8; 7,55-7,6 |
| 26 | 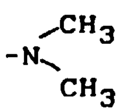 | $CH_3$ | 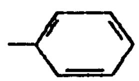 | CN | $CH_3$ | $-CH_2-CH_2-$ | O | $^{1}H$-NMR*): 1,85; 2,23-2,5; 2,9; 3,8; 7,18-7,32 |

| Bsp.-Nr. | 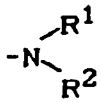 | $R^3$ | $R^4$ | $R^5$ | $R^6$ | A | X | physikalische Eigenschaften |
|---|---|---|---|---|---|---|---|---|
| 27 | 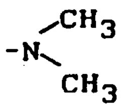 | $CH_3$ | 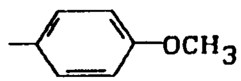 | CN | $CH_3$ | $-CH_2-CH_2-$ | O | Fp.: 97-99° C |
| 28 | 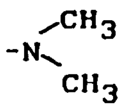 | $CH_3$ | 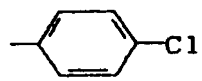 | H | $CH_3$ | $-CH_2-CH_2-$ | O | ¹H-NMR*): 1,25-1,28; 2,62-2,7; 2,9; 3,8; 4,08-4,2 |
| 29 | 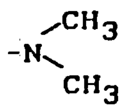 | $CH_3$ | 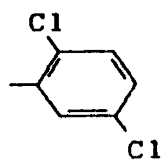 | $CH_3$ | $CH_3$ | $-CH_2-CH_2-$ | O | Fp.: 111-113° C |
| 31 | 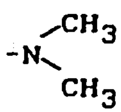 | $CH_3$ | 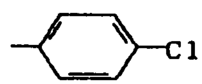 | $CH_3$ | $CH_3$ | $-CH_2-CH_2-$ | O | ¹H-NMR*): 1,46; 2,9; 3,76 |
| 32 | 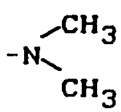 | $CH_3$ | 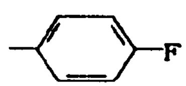 | $CH_3$ | $CH_3$ | $-CH_2-CH_2-$ | O | ¹H-NMR*): 1,47; 2,9; 3,77 |

| Bsp.-Nr. | 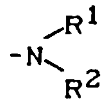 | $R^3$ | $R^4$ | $R^5$ | $R^6$ | A | X | physikalische Eigenschaften |
|---|---|---|---|---|---|---|---|---|
| 35 | 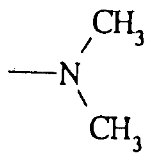 | $CH_3$ | 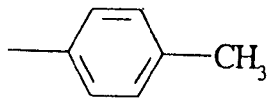 | $CH_3$ | $CH_3$ | $—CH_2–CH_2—$ | O | $^1$H-NMR*): 1,47; 2,29; 2,89; 3,76 |
| 36 | 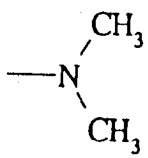 | $CH_3$ | 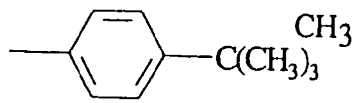 | $CH_3$ | $CH_3$ | $—CH_2–CH_2—$ | O | $^1$H-NMR*): 1,29; 1,47; 2,89; 3,77 |
| 37 | 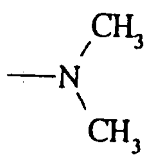 | $CH_3$ | 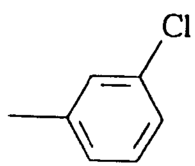 | H | $CH_3$ | $—CH_2–O—$ | O | $^1$H-NMR*): 1,40-1,43; 2,9; 3,8; 4,0-4,03; 4,45-4,55 |

| Bsp.-Nr. | 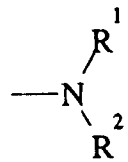 | $R^3$ | $R^4$ | $R^5$ | $R^6$ | A | X | physikalische Eigenschaften |
|---|---|---|---|---|---|---|---|---|
| 38 | 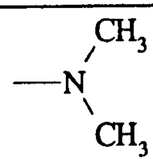 | $CH_3$ | 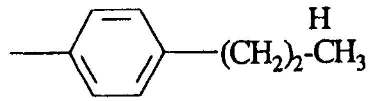 | H | $CH_3$ | $-CH_2-CH_2-$ | O | $^1H$-NMR*): 0,9-0,95; 1,25-1,3; 1,65-1,75; 2,5-2,55; 2,6-2,7; 3,03; 3,85; 4,1-4,2 |
| 39 | 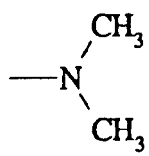 | $CH_3$ | 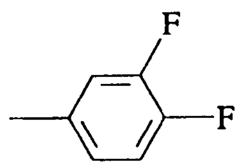 | H | $CH_3$ | $-CH_2-CH_2-$ | O | $^1H$-NMR*): 1,17-1,2; 2,5-2,6; 2,85; 3,73 |

NMR

*) Die $^1H$-NMR-Spektren wurden in Deuterochloroform ($CDCl_3$) oder Hexadeuterodimethylsulfoxid (DMSO-$d_6$) mit Tetramethylsilan (TMS) als innerem Standard aufgenommen. Angegeben ist die chemische Verschiebung als δ-Wert in ppm.

Anwendungsbeispiele:

In dem folgenden Anwendungsbeispiel wurde die nachstehend aufgeführte Verbindung als Vergleichssubstanz eingesetzt:

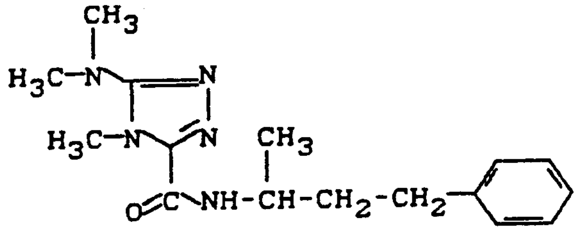

(A)

5-Dimethylamino-4-methyl-3-(4-phenylbut-2-ylaminocarbonyl)-1,2,4-triazol (bekannt aus DE 38 09 053).

Beispiel A:

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden.

Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele: 3, 4, 5, 6, 7, 9, 11, 12, 13, 14 und 19.

## Patentansprüche

### Patentansprüche für folgende Vertragsstaaten : BE, CH, DE, DK, FR, GB, IT, LI, NL

**1.** Substituierte Triazole der allgemeinen Formel (I)

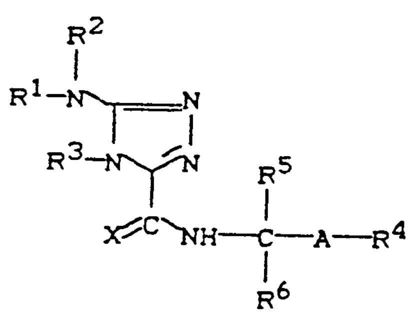

(I)

bei welchen

$R^1$ für geradkettiges oder verzweigtes Alkyl mit 1 bis 6 Kohlenstoffatomen steht,
$R^2$ für geradkettiges oder verzweigtes Alkyl mit 1 bis 6 Kohlenstoffatomen steht,
$R^3$ für geradkettiges oder verzweigtes Alkyl mit 1 bis 6 Kohlenstoffatomen steht,
$R^4$ für gegebenenfalls einfach oder mehrfach, gleich oder verschieden substituiertes Aryl mit 6 oder 10 Kohlenstoffatomen steht, wobei als Substituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Dialkylamino mit jeweils 1 bis 4 Kohlenstoffatomen in den einzelnen geradkettigen oder verzweigten Alkylteilen, N-Alkanoylamino mit 1 bis 5 Kohlenstoffatomen im geradkettigen oder verzweigten Alkanoylteil, gegebenenfalls einfach oder mehrfach, gleich oder verschieden durch Halogen substituiertes, zweifach verknüpftes Dioxyalkylen mit 1 bis Kohlenstoffatomen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl, Phenoxy, α- oder β-Naphthyl, und außerdem $R^4$ für Indanyl steht;
$R^5$ entweder für Wasserstoff, für geradkettiges oder verzweigtes Alkyl mit 1 bis 6 Kohlenstoffatomen oder für Cyano steht und
$R^6$ für Wasserstoff oder für geradkettiges oder verzweigtes Alkyl mit 1 bis 6 Kohlenstoffatomen steht oder
$R^5$ und $R^6$ gemeinsam für zweifach verknüpftes Alkandiyl mit 2 bis 9 Kohlenstoffatomen stehen,
A für einen Rest der Formel $-CH_2-CH_2-$; $-CH(CH_3)-CH_2-$; $-CH_2-O-$ steht und
X für Sauerstoff oder Schwefel steht,

wobei jedoch die Verbindung 5-Dimethylamino-4-methyl-3-(4-phenylbut-2-ylaminocarbonyl)-1,2,4-triazol ausgenommen ist.

2. Substituierte Triazole der allgemeinen Formel (I) gemäß Anspruch 1, bei welchen
$R^1$ für geradkettiges oder verzweigtes Alkyl mit 1 bis 4 Kohlenstoffatomen steht,
$R^2$ für geradkettiges oder verzweigtes Alkyl mit 1 bis 4 Kohlenstoffatomen steht,
$R^3$ für geradkettiges oder verzweigtes Alkyl mit 1 bis 4 Kohlenstoffatomen steht,
$R^4$ für jeweils gegebenenfalls ein- bis dreifach, gleich oder verschieden substituiertes Phenyl, α-Naphthyl, β-Naphthyl oder Indanyl, steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Difluormethyl, Trifluormethyl, Difluormethoxy, Trifluormethoxy, Difluormethylthio, Trifluormethylthio, Dimethylamino, Diethylamino, N-Acetamido, Dioxymethylen, Difluordioxymethylen, Dioxyethylen, Trifluordioxyethylen, Tetrafluordioxyethylen, Methoxycarbonyl, Ethoxycarbonyl, Propoxycarbonyl, Methoximinomethyl, Methoximinoethyl, Ethoximinomethyl, Ethoximinoethyl oder jeweils gegebenenfalls ein-bis dreifach, gleich oder verschieden durch Fluor, Chlor, Brom, Methyl, Ethyl, Methoxy und/oder Ethoxy substituiertes Phenyl, Phenoxy, α-Naphthyl oder β-Naphthyl,
$R^5$ entweder für Wasserstoff, für geradkettiges oder verzweigtes Alkyl mit 1 bis 4 Kohlenstoffatomen oder für Cyano steht und
$R^6$ für Wasserstoff oder für geradkettiges oder verzweigtes Alkyl mit 1 bis 4 Kohlenstoffatomen steht oder
$R^5$ und $R^6$ gemeinsam für zweifach verknüpftes Alkandiyl mit 4 bis 9 Kohlenstoffatomen stehen,
A für einen der Reste $-CH_2-CH_2-$; $-CH(CH_3)-CH_2-$; $CH_2-O-$;
steht und
X für Sauerstoff oder Schwefel steht, wobei jedoch die Verbindung 5-Dimethylamino-4-methyl-3-(4-phenylbut-2-ylaminocarbonyl)-1,2,4-triazol ausgenommen ist.

3. Substituierte Triazole der allgemeinen Formel (I) gemäß Anspruch 1, bei welchen
$R^1$ für Methyl steht,
$R^2$ für Methyl oder Ethyl steht,
$R^3$ für Methyl oder Ethyl steht,

| | |
|---|---|
| $R^4$ | für gegebenenfalls ein- bis dreifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Difluormethyl, Trifluormethyl, Difluormethoxy, Trifluormethoxy, Difluormethylthio, Trifluormethylthio, Dimethylamino, Diethylamino, N-Acetamido, Dioxymethylen, Difluordioxymethylen, Dioxyethylen, Trifluordioxyethylen, Tetrafluordioxyethylen, Methoxycarbonyl, Ethoxycarbonyl, Propoxycarbonyl, Methoximinomethyl, Methoximinoethyl, Ethoximinomethyl, Ethoximinoethyl oder jeweils gegebenenfalls ein- bis dreifach, gleich oder verschieden durch Fluor, Chlor, Brom, Methyl, Ethyl, Methoxy und/oder Ethoxy substituiertes Phenyl, Phenoxy, α-Naphthyl oder β-Naphthyl, |
| $R^5$ | für Wasserstoff, Methyl, Ethyl oder Cyano steht, |
| $R^6$ | für Methyl, Ethyl, n- oder i-Propyl steht, |
| A | für einen der Reste $-CH_2-CH_2-$; $-CH(CH_3)-CH_2-$; $CH_2-O-$; steht und |
| X | für Sauerstoff steht, |

wobei jedoch die Verbindung 5-Dimethylamino-4-methyl-3-(4-phenylbut-2-ylaminocarbonyl)-1,2,4-triazol ausgenommen ist.

4. Verfahren zur Herstellung von substituierten Triazolen der allgemeinen Formel (I) gemäß Anspruch 1

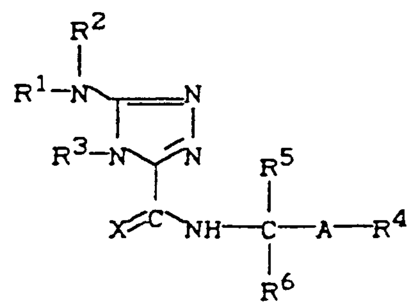

(I)

bei welchen

| | |
|---|---|
| $R^1$ | für geradkettiges oder verzweigtes Alkyl mit 1 bis 6 Kohlenstoffatomen steht, |
| $R^2$ | für geradkettiges oder verzweigtes Alkyl mit 1 bis 6 Kohlenstoffatomen steht, |
| $R^3$ | für geradkettiges oder verzweigtes Alkyl mit 1 bis 6 Kohlenstoffatomen steht, |
| $R^4$ | für gegebenenfalls einfach oder mehrfach, gleich oder verschieden substituiertes Aryl mit 6 oder 10 Kohlenstoffatomen steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Dialkylamino mit jeweils 1 bis 4 Kohlenstoffatomen in den einzelnen geradkettigen oder verzweigten Alkylteilen, N-Alkanoylamino mit 1 bis 5 Kohlenstoffatomen im geradkettigen oder verzweigten Alkanoylteil, gegebenenfalls einfach oder mehrfach, gleich oder verschieden durch Halogen substituiertes, zweifach verknüpftes Dioxyalkylen mit 1 bis 3 Kohlenstoffatomen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl, Phenoxy, α- oder β-Naphthyl und außerdem $R^4$ für Indanyl steht; |
| $R^5$ | entweder für Wasserstoff, für geradkettiges oder verzweigtes Alkyl mit 1 bis 6 Kohlenstoffatomen oder für Cyano steht und |
| $R^6$ | für Wasserstoff oder für geradkettiges oder verzweigtes Alkyl mit 1 bis 6 Kohlenstoffatomen steht oder |
| $R^5$ und $R^6$ | gemeinsam für zweifach verknüpftes Alkandiyl mit 2 bis 9 Kohlenstoffatomen stehen, |
| A | für einen Rest der Formel $-CH_2-CH_2-$; $-CH(CH_3)-CH_2-$; $-CH_2-O-$ steht und |

X für Sauerstoff oder Schwefel steht,

wobei jedoch die Verbindung 5-Dimethylamino-4-methyl-3-(4-phenylbut-2-ylaminocarbonyl)-1,2,4-triazol ausgenommen ist,

dadurch gekennzeichnet, daß man

a) Aminoguanidine der Formel (II),

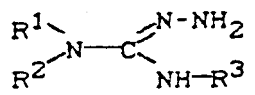

(II)

in welcher

$R^1$, $R^2$ und $R^3$ die oben angegebene Bedeutung haben,

oder deren Säureadditionssalze mit (Thio)Oxalesteramiden der Formel (III),

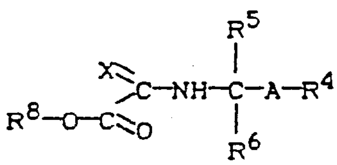

(III)

in welcher

$R^4$, $R^5$, $R^6$, X und A die oben angegebene Bedeutung haben und

$R^8$ für geradkettiges oder verzweigtes Alkyl mit 1 bis 4 Kohlenstoffatomen steht,

gegebenenfalls in Gegenwart eines Verdünnungsmittels und gegebenenfalls in Gegenwart eines Reaktionshifsmittels umsetzt,

oder daß man

b) substituierte Triazolyl(thio)carbonsäureester der Formel (IV),

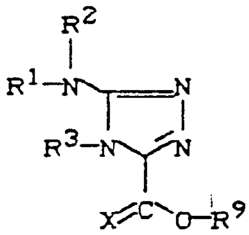

(IV)

in welcher

$R^1$, $R^2$, $R^3$ und X die oben angegebene Bedeutung haben und

$R^9$ für geradkettiges oder verzweigtes Alkyl mit 1 bis 4 Kohlenstoffatomen steht,

mit Aminen der Formel (V),

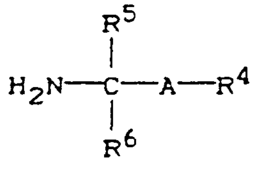

(V)

in welcher

$R^4$, $R^5$, $R^6$ und A die oben angegebene Bedeutung haben,

gegebenenfalls in Gegenwart eines Verdünnungsmittels umsetzt.

5. Herbizide Mittel, gekennzeichnet durch einen Gehalt an mindestens einem substituierten Triazol der Formel (I) gemäß den Ansprüchen 1 bis 4.

6. Verfahren zur Bekämpfung von unerwünschten Pflanzen, dadurch gekennzeichnet, daß man substituierte Triazole der Formel (I) gemäß den Ansprüchen 1 bis 4 auf unerwünschte Pflanzen und/oder ihren Lebensraum einwirken läßt.

7. Verwendung von substituierten Triazolen der Formel (I) gemäß den Ansprüchen 1 bis 4 zur Bekämpfung von unerwünschten Pflanzen.

8. Verfahren zur Herstellung von herbiziden Mitteln, dadurch gekennzeichnet, daß man substituierte Triazole der Formel (I) gemaß den Ansprüchen 1 bis 4 mit Streckmitteln und/oder oberflächenaktiven Substanzen vermischt.

**Patentansprüche für folgenden Vertragsstaat : ES**

1. Verfahren zur Herstellung von substituierten Triazolen der allgemeinen Formel (I)

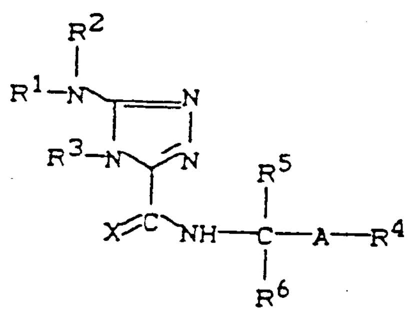

(I)

bei welchen

| | |
|---|---|
| $R^1$ | für geradkettiges oder verzweigtes Alkyl mit 1 bis 6 Kohlenstoffatomen steht, |
| $R^2$ | für geradkettiges oder verzweigtes Alkyl mit 1 bis 6 Kohlenstoffatomen steht, |
| $R^3$ | für geradkettiges oder verzweigtes Alkyl mit 1 bis 6 Kohlenstoffatomen steht, |
| $R^4$ | für gegebenenfalls einfach oder mehrfach, gleich oder verschieden substituiertes Aryl mit 6 oder 10 Kohlenstoffatomen steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Dialkylamino mit jeweils 1 bis 4 Kohlenstoffatomen in den einzelnen geradkettigen oder verzweigten Alkylteilen, N-Alkanoylamino mit 1 bis 5 Kohlenstoffatomen im geradkettigen oder verzweigten Alkanoylteil, gegebenenfalls einfach oder mehrfach, gleich oder verschieden durch Halogen substituiertes, zweifach verknüpftes Dioxyalkylen mit 1 bis 3 Kohlenstoffatomen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl, Phenoxy, α- oder β-Naphthyl, und außerdem $R^4$ für Indanyl steht; |
| $R^5$ | entweder für Wasserstoff, für geradkettiges oder verzweigtes Alkyl mit 1 bis 6 Kohlenstoffatomen oder für Cyano steht und |
| $R^6$ | für Wasserstoff oder für geradkettiges oder verzweigtes Alkyl mit 1 bis 6 Kohlenstoffatomen steht oder |
| $R^5$ und $R^6$ | gemeinsam für zweifach verknüpftes Alkandiyl mit 2 bis 9 Kohlenstoffatomen stehen, |
| A | für einen Rest der Formel $-CH_2-CH_2-$; $-CH(CH_3)-CH_2-$; $-CH_2-O-$ steht und |
| X | für Sauerstoff oder Schwefel steht, wobei |

wobei jedoch die Verbindung 5-Dimethylamino-4-methyl-3-(4-phenylbut-2-ylam;nocarbonyl)-1,2,d-triazol ausgenommen ist,
dadurch gekennzeichnet, daß man

a) Aminoguanidine der Formel (II),

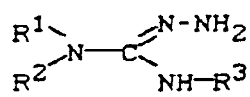

(II)

in welcher
$R^1$, $R^2$ und $R^3$ die oben angegebene Bedeutung haben,
oder deren Säureadditionssalze mit (Thio)Oxalesteramiden der Formel (III),

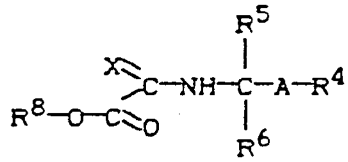

(III)

in welcher

| | |
|---|---|
| $R^4$, $R^5$, $R^6$, X und A | die oben angegebene Bedeutung haben und |
| $R^8$ | für geradkettiges oder verzweigtes Alkyl mit 1 bis 4 Kohlenstoffatomen steht, |

gegebenenfalls in Gegenwart eines Verdünnungsmittels und gegebenenfalls in Gegenwart eines Reaktionshilfsmittels umsetzt,
oder daß man

b) substituierte Triazolyl(thio)carbonsäureester der Formel (IV),

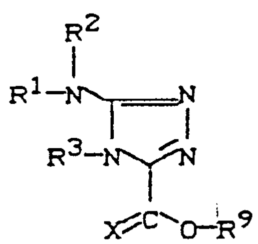

(IV)

in welcher

| | |
|---|---|
| $R^1$, $R^2$, $R^3$ und X | die oben angegebene Bedeutung haben und |
| $R^9$ | für geradkettiges oder verzweigtes Alkyl mit 1 bis 4 Kohlenstoffatomen steht, |
| | mit Aminen der Formel (V), |

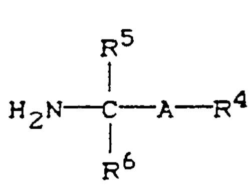

(V)

in welcher

| | |
|---|---|
| $R^4$, $R^5$, $R^6$ und A | die oben angegebene Bedeutung haben, |

gegebenenfalls in Gegenwart eines Verdünnungsmittels umsetzt.

2. Verfahren zur Herstellung von substituierten Triazolen der allgemeinen Formel (I) gemäß Anspruch 1 bei

welchen

$R^1$ für geradkettiges oder verzweigtes Alkyl mit 1 bis 4 Kohlenstoffatomen steht,

$R^2$ für geradkettiges oder verzweigtes Alkyl mit 1 bis 4 Kohlenstoffatomen steht,

$R^3$ für geradkettiges oder verzweigtes Alkyl mit 1 bis 4 Kohlenstoffatomen steht,

$R^4$ für jeweils gegebenenfalls ein- bis dreifach, gleich oder verschieden substituiertes Phenyl, α-Naphthyl, β-Naphthyl oder Inda-nyl, steht, wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Difluormethyl, Trifluormethyl, Difluormethoxy, Trifluormethoxy, Difluormethylthio, Trifluormethylthio, Dimethylamino, Diethylamino, N-Acetamido, Dioxymethylen, Difluordioxymethylen, Dioxyethylen, Trifluordioxyethylen, Tetrafluordioxyethylen, Methoxycartonyl, Ethoxycarbonyl, Propoxycarbonyl, Methoximinomethyl, Methoximinoethyl, Ethoximinomethyl, Ethoximinoethyl oder jeweils gegebenenfalls ein- bis dreifach, gleich oder verschieden durch Fluor, Chlor, Brom, Methyl, Ethyl, Methoxy und/oder Ethoxy substituiertes Phenyl, Phenoxy, α-Naphthyl oder β-Naphthyl,

$R^5$ entweder für Wasserstoff, für geradkettiges oder verzweigtes Alkyl mit 1 bis 4 Kohlenstoffatomen oder für Cyano steht und

$R^6$ für Wasserstoff oder für geradkettiges oder verzweigtes Alkyl mit 1 bis 4 Kohlenstoffatomen steht oder

$R^5$ und $R^6$ gemeinsam für zweifach verknüpftes Alkandiyl mit 4 bis 9 Kohlenstoffatomen stehen,

A für einen der Reste $-CH_2-CH_2-$; $-CH(CH_3)-CH_2-$; $CH_2-O-$ steht und

X für Sauerstoff oder Schwefel steht, wobei jedoch die Verbindung 5-Dimethylamino-4-methyl-3-(4-phenylbut-2-ylaminocarbonyl)-1,2,4-triazol ausgenommen ist.

**3.** Verfahren zur Herstellung von substituierten Triazolen der allgemeinen Formel (I) gemäß Anspruch 1 bei welchen

$R^1$ für Methyl steht,

$R^2$ für Methyl oder Ethyl steht,

$R^3$ für Methyl oder Ethyl steht,

$R^4$ für gegebenenfalls ein- bis dreifach, gleich oder verschieden substituiertes Phenyl steht, wobei als Substituenten infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Difluormethyl, Trifluormethyl, Difluormethoxy, Trifluormethoxy, Difluormethylthio, Trifluormethylthio, Dimethylamino, Diethylamino, N-Acetamido, Dioxymethylen, Difluordioxymethylen, Dioxyethylen, Trifluordioxyethylen, Tetrafluordioxyethylen, Methoxycarbonyl, Ethoxycarbonyl, Propoxycarbonyl, Methoximinomethyl, Methoximinoethyl, Ethoximinomethyl, Ethoximinoethyl oder jeweils gegebenenfalls ein- bis dreifach, gleich oder verschieden durch Fluor, Chlor, Brom, Methyl, Ethyl, Methoxy und/oder Ethoxy substituiertes Phenyl, Phenoxy, α- oder β-Naphthyl,

$R^5$ für Wasserstoff, Methyl, Ethyl oder Cyano steht,

$R^6$ für Methyl, Ethyl, n- oder i-Propyl steht,

A für einen der Reste $-CH_2-CH_2-$; $-CH(CH_3)-CH_2-$; $CH_2-O-$ steht und

X für Sauerstoff steht,

wobei jedoch die Verbindung 5-Dimethylamino-4-methyl-3-(4-phenylbut-2-ylaminocarbonyl)-1,2,4-triazol ausgenommen ist.

**4.** Herbizide Mittel, gekennzeichnet durch einen Gehalt an mindestens einem substituierten Triazol der Formel (I) gemäß den Ansprüchen 1 bis 3.

**5.** Verfahren zur Bekämpfung von unerwünschten Pflanzen, dadurch gekennzeichnet, daß man substituierte Triazole der Formel (I) gemäß den Ansprüchen 1 bis 3 auf unerwünschte Pflanzen und/oder ihren Lebensraum einwirken läßt.

**6.** Verwendung von substituierten Triazolen der Formel (I) gemäß den Ansprüchen 1 bis 3 zur Bekämpfung von unerwünschten Pflanzen.

7. Verfahren zur Herstellung von herbiziden Mitteln, dadurch gekennzeichnet, daß man substituierte Triazole der Formel (I) gemäß den Ansprüchen 1 bis 3 mit Streckmitteln und/oder oberflächenaktiven Substanzen vermischt.

## Claims

### Claims for the following Contracting States : BE, CH, DE, DK, FR, GB, IT, LI, NL

1. Substituted triazoles of the general formula (I)

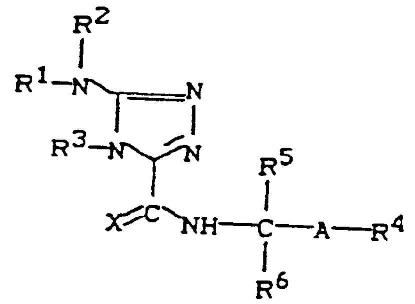

(I)

in which

| | |
|---|---|
| $R^1$ | represents straight-chain or branched alkyl having 1 to 6 carbon atoms, |
| $R^2$ | represents straight-chain or branched alkyl having 1 to 6 carbon atoms, |
| $R^3$ | represents straight-chain or branched alkyl having 1 to 6 carbon atoms, |
| $R^4$ | represents aryl which has 6 or 10 carbon atoms and which is optionally monosubstituted or polysubstituted by identical or different substituents, suitable substituents being: halogen, cyano, nitro, in each case straight-chain or branched alkyl, alkoxy or alkylthio, each of which has 1 to 4 carbon atoms, in each case straight-chain or branched halogenoalkyl, halogenoalkoxy, halogenoalkylthio, each of which has 1 to 4 carbon atoms and 1 to 9 identical or different halogen atoms, in each case straight-chain or branched alkoxycarbonyl or alkoximinoalkyl, each of which has 1 to 4 carbon atoms in the individual alkyl moieties, dialkylamino having in each case 1 to 4 carbon atoms in the individual straight-chain or branched alkyl moieties, N-alkanoylamino having 1 to 5 carbon atoms in the straight-chain or branched alkanolyl moiety, divalent dioxyalkylene which has 1 to 3 carbon atoms and which is optionally monosubsti-tuted or polysubstituted by identical or different halogen substituents, and phenyl, phenoxy, α-naphthyl or β-naphthyl, each of which is optionally monosubstituted or polysubstituted by identical or different substituents from the series comprising halogen and/or straight-chain or branched alkyl having 1 to 4 carbon atoms, and furthermore $R^4$ represents indanyl; |
| $R^5$ | either represents hydrogen, straight-chain or branched alkyl having 1 to 6 carbon atoms or cyano and |
| $R^6$ | represents hydrogen or straight-chain or branched alkyl having 1 to 6 carbon atoms or |
| $R^5$ and $R^6$ | together represent divalent alkanediyl having 2 to 9 carbon atoms, |
| A | represents a radical of the formula $-CH_2-CH_2-$; $-CH(CH_3)-CH_2-$ or $-CH_2-O-$ and |
| X | represents oxygen or sulphur, but with the exception of the compound 5-dimethylamino-4-methyl-3-(4-phenylbut-2-ylaminocarbonyl)-1,2,4-triazole. |

2. Substituted triazoles of the general formula (I) according to Claim 1, in which

| | |
|---|---|
| $R^1$ | represents straight-chain or branched alkyl having 1 to 4 carbon atoms, |
| $R^2$ | represents straight-chain or branched alkyl having 1 to 4 carbon atoms, |
| $R^3$ | represents straight-chain or branched alkyl having 1 to 4 carbon atoms, |
| $R^4$ | represents phenyl, α-naphthyl, β-naphthyl or indanyl, each of which is optionally monosubstituted to trisubstituted by identical or different substituents, suitable substituents in each case being: fluorine, chlorine, bromine, cyano, nitro, methyl, ethyl, n- or i-propyl, n-, i-, s- or t-butyl, methoxy, ethoxy, n- or i-propoxy, n-, i-, s- or t-butoxy, methylthio, ethylthio, difluoromethyl, trifluoromethyl, difluoromethoxy, trifluoromethoxy, difluoro- |

methylthio, trifluoromethylthio, dimethylamino, diethylamino, N-acetamido, dioxymethylene, difluorodioxymethylene, dioxyethylene, trifluorodioxyethylene, tetrafluorodioxyethylene, methoxycarbonyl, ethoxycarbonyl, propoxycarbonyl, methoximinomethyl, methoximinoethyl, ethoximinomethyl, ethoximinoethyl, or phenyl, phenoxy, α-naphthyl or β-naphthyl, each of which is optionally monosubstituted to trisubstituted by identical or different substituents from the series comprising fluorine, chlorine, bromine, methyl, ethyl, methoxy and/or ethoxy,

$R^5$ either represents hydrogen, straight-chain or branched alkyl having 1 to 4 carbon atoms or cyano and

$R^6$ represents hydrogen or straight-chain or branched alkyl having 1 to 4 carbon atoms or

$R^5$ and $R^6$ together represent divalent alkanediyl having 4 to 9 carbon atoms,

A represents one of the radicals $-CH_2-CH_2-$; $-CH(CH_3)-CH_2-$ or $-CH_2-O-$ and

X represents oxygen or sulphur,

but with the exception of the compound 5-dimethylamino-4-methyl-3-(4-phenylbut-2-ylaminocarbonyl)-1,2,4-triazole.

3. Substituted triazoles of the general formula (I) according to Claim 1, in which

$R^1$ represents methyl,

$R^2$ represents methyl or ethyl,

$R^3$ represents methyl or ethyl,

$R^4$ represents phenyl which is optionally monosubstituted to trisubstituted by identical or different substituents, suitable substituents being:
fluorine, chlorine, bromine, cyano, nitro, methyl, ethyl, n- or i-propyl, n-, i-, s- or t-butyl, methoxy, ethoxy, n- or i-propoxy, n-, i-, s- or t-butoxy, methylthio, ethylthio, difluoromethyl, trifluoromethyl, difluoromethoxy, trifluoromethoxy, difluoromethylthio, trifluoromethylthio, dimethylamino, diethylamino, N-acetamido, dioxymethylene, difluorodioxymethylene, dioxyethylene, trifluorodioxyethylene, tetrafluorodioxyethylene, methoxycarbonyl, ethoxycarbonyl, propoxycarbonyl, methoximinomethyl, methoximinoethyl, ethoximinomethyl, ethoximinoethyl, or phenyl, phenoxy, α-naphthyl or β-naphthyl, each of which is optionally monosubstituted to trisubstituted by identical or different substituents from the series comprising fluorine, chlorine, bromine, methyl, ethyl, methoxy and/or ethoxy,

$R^5$ represents hydrogen, methyl, ethyl or cyano,

$R^6$ represents methyl, ethyl, n- or i-propyl,

A represents one of the radicals $-CH_2-CH_2-$; $-CH(CH_3)-CH_2-$ or $-CH_2-O-$ and

X represents oxygen,

but with the exception of the compound 5-dimethylamino-4-methyl-3-(4-phenylbut-2-ylaminocarbonyl)-1,2,4-triazole.

4. Process for the preparation of substituted triazoles of the general formula (I) according to Claim 1

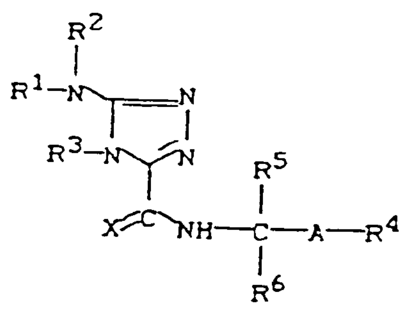

(I)

in which

$R^1$ represents straight-chain or branched alkyl having 1 to 6 carbon atoms,

$R^2$ represents straight-chain or branched alkyl having 1 to 6 carbon atoms,

$R^3$ represents straight-chain or branched alkyl having 1 to 6 carbon atoms,

$R^4$ represents aryl which has 6 or 10 carbon atoms and which is optionally monosubstituted or polysubstituted by identical or different substituents, suitable substituents being:
halogen, cyano, nitro, in each case straight-chain or branched alkyl, alkoxy or alkylthio,

each of which has 1 to 4 carbon atoms, in each case straight-chain or branched halogenoalkyl, halogenoalkoxy, halogenoalkylthio, each of which has 1 to 4 carbon atoms and 1 to 9 identical or different halogen atoms, in each case straight-chain or branched alkoxycarbonyl or alkoximinoalkyl, each of which has 1 to 4 carbon atoms in the individual alkyl moieties, dialkylamino having in each case 1 to 4 carbon atoms in the individual straight-chain or branched alkyl moieties, N-alkanoylamino having 1 to 5 carbon atoms in the straight-chain or branched alkanolyl moiety, divalent dioxyalkylene which has 1 to 3 carbon atoms and which is optionally monosubstituted or polysubstituted by identical or different halogen substituents, and phenyl, phenoxy, α-naphthyl or β-naphthyl, each of which is optionally monosubstituted or polysubstituted by identical or different substituents from the series comprising halogen and/or straight-chain or branched alkyl having 1 to 4 carbon atoms, and furthermore $R^4$ represents indanyl;

$R^5$ either represents hydrogen, straight-chain or branched alkyl having 1 to 6 carbon atoms or cyano and

$R^6$ represents hydrogen or straight-chain or branched alkyl having 1 to 6 carbon atoms or

$R^5$ and $R^6$ together represent divalent alkanediyl having 2 to 9 carbon atoms,

A represents a radical of the formula $-CH_2-CH_2-$; $-CH(CH_3)-CH_2-$ or $-CH_2-O-$ and

X represents oxygen or sulphur,

but with the exception of the compound 5-dimethylamino-4-methyl-3-(4-phenylbut-2-ylaminocarbonyl)-1,2,4-triazole,

characterized in that

a) aminoguanidines of the formula (II)

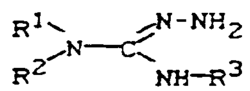

(II)

in which

$R^1$, $R^2$ and $R^3$ have the abovementioned meaning,

or their acid addition salts are reacted with (thio)oxamates of the formula (III)

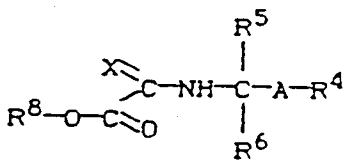

(III)

in which

$R^4$, $R^5$, $R^6$, X and A have the abovementioned meaning and

$R^8$ represents straight-chain or branched alkyl having 1 to 4 carbon atoms,

if appropriate in the presence of a diluent and if appropriate in the presence of a reaction auxiliary, or in that

b) substituted triazolyl(thio)carboxylates of the formula (IV)

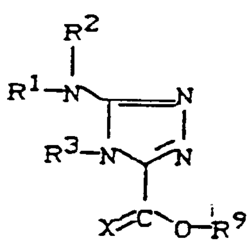

(IV)

in which

$R^1$, $R^2$, $R^3$ and X have the abovementioned meaning and

$R^9$ represents straight-chain or branched alkyl having 1 to 4 carbon atoms,
are reacted with amines of the formula (V)

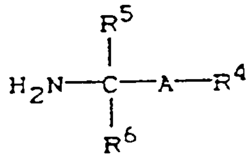

(V)

in which
$R^4$, $R^5$, $R^6$ and A have the abovementioned meaning,
if appropriate in the presence of a diluent.

5. Herbicidal agents, characterized in that they contain at least one substituted triazole of the formula (I) according to Claims 1 to 4.

6. Process for combating undesired plants, characterized in that substituted triazoles of the formula (I) according to Claims 1 to 4 are allowed to act on undesired plants and/or their environment.

7. Use of substituted triazoles of the formula (I) according to Claims 1 to 4 for combating undesired plants.

8. Process for the preparation of herbicidal agents, characterized in that substituted triazoles of the formula (I) according to Claims 1 to 4 are mixed with extenders and/or surface-active substances.

**Claims for the following Contracting State : ES**

1. Process for the preparation of substituted triazoles of the general formula (I)

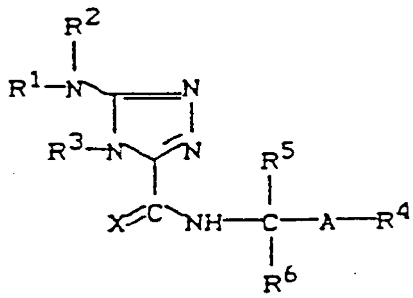

(I)

in which
$R^1$ represents straight-chain or branched alkyl having 1 to 6 carbon atoms,
$R^2$ represents straight-chain or branched alkyl having 1 to 6 carbon atoms,
$R^3$ represents straight-chain or branched alkyl having 1 to 6 carbon atoms,
$R^4$ represents aryl which has 6 or 10 carbon atoms and which is optionally monosubstituted or polysubstituted by identical or different substituents, suitable substituents being: halogen, cyano, nitro, in each case straight-chain or branched alkyl, alkoxy or alkylthio, each of which has 1 to 4 carbon atoms, in each case straight-chain or branched halogenoalkyl, halogenoalkoxy, halogenoalkylthio, each of which has 1 to 4 carbon atoms and 1 to 9 identical or different halogen atoms, in each case straight-chain or branched alkoxycarbonyl or alkoximinoalkyl, each of which has 1 to 4 carbon atoms in the individual alkyl moieties, dialkylamino having in each case 1 to 4 carbon atoms in the individual straight-chain or branched alkyl moieties, N-alkanoylamino having 1 to 5 carbon atoms in the straight-chain or branched alkanolyl moiety, divalent dioxyalkylene which has 1 to 3 carbon atoms and which is optionally monosubstituted or polysubstituted by identical or different halogen substituents, and phenyl, phenoxy, α-naphthyl or β-naphthyl, each of which is optionally monosubstituted or polysubstituted by identical or different substituents from the series comprising halogen and/or straight-chain or branched alkyl having 1 to 4 carbon atoms, and furthermore $R^4$ represents indanyl;

$R^5$ either represents hydrogen, straight-chain or branched alkyl having 1 to 6 carbon atoms or cyano and

$R^6$ represents hydrogen or straight-chain or branched alkyl having 1 to 6 carbon atoms or

$R^5$ and $R^6$ together represent divalent alkanediyl having 2 to 9 carbon atoms,

A represents a radical of the formula $-CH_2-CH_2-$; $-CH(CH_3)-CH_2-$ or $-CH_2-O-$ and

X represents oxygen or sulphur, but with the exception of the compound 5-dimethylamino-4-methyl-3-(4-phenylbut-2-ylaminocarbonyl)-1,2,4-triazole,

characterized in that

a) aminoguanidines of the formula (II)

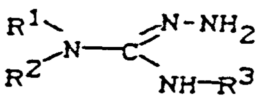

(II)

in which

$R^1$, $R^2$ and $R^3$ have the abovementioned meaning,

or their acid addition salts are reacted with (thio)oxamates of the formula (III)

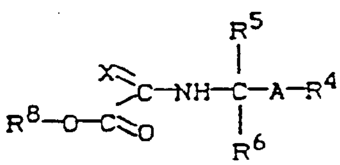

(III)

in which

$R^4$, $R^5$, $R^6$, X and A have the abovementioned meaning and

$R^8$ represents straight-chain or branched alkyl having 1 to 4 carbon atoms,

if appropriate in the presence of a diluent and if appropriate in the presence of a reaction auxiliary, or in that

b) substituted triazolyl(thio)carboxylates of the formula (IV)

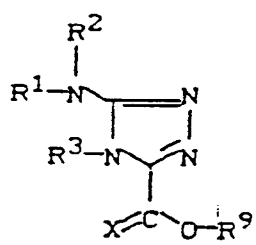

(IV)

in which

$R^1$, $R^2$, $R^3$ and X have the abovementioned meaning and

$R^9$ represents straight-chain or branched alkyl having 1 to 4 carbon atoms,

are reacted with amines of the formula (V)

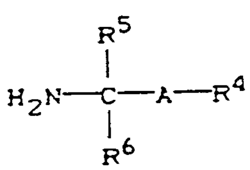

(V)

in which

$R^4$, $R^5$, $R^6$ and A have the abovementioned meaning, if appropriate in the presence of a diluent.

2. Process for the preparation of substituted triazoles of the general formula (I) according to Claim 1, in which

| | |
|---|---|
| $R^1$ | represents straight-chain or branched alkyl having 1 to 4 carbon atoms, |
| $R^2$ | represents straight-chain or branched alkyl having 1 to 4 carbon atoms, |
| $R^3$ | represents straight-chain or branched alkyl having 1 to 4 carbon atoms, |
| $R^4$ | represents phenyl, α-naphthyl, β-naphthyl or indanyl, each of which is optionally monosubstituted to trisubstituted by identical or different substituents, suitable substituents in each case being: fluorine, chlorine, bromine, cyano, nitro, methyl, ethyl, n- or i-propyl, n-, i-, s- or t-butyl, methoxy, ethoxy, n- or i-propoxy, n-, i-, s- or t-butoxy, methylthio, ethylthio, difluoromethyl, trifluoromethyl, difluoromethoxy, trifluoromethoxy, difluoromethylthio, trifluoromethylthio, dimethylamino, diethylamino, N-acetamido, dioxymethylene, difluorodioxymethylene, dioxyethylene, trifluorodioxyethylene, tetrafluorodioxyethylene, methoxycarbonyl, ethoxycarbonyl, propoxycarbonyl, methoximinomethyl, methoximinoethyl, ethoximinomethyl, ethoximinoethyl, or phenyl, phenoxy, α-naphthyl or β-naphthyl, each of which is optionally mono-substituted to trisubstituted by identical or different substituents from the series comprising fluorine, chlorine, bromine, methyl, ethyl, methoxy and/or ethoxy, |
| $R^5$ | either represents hydrogen, straight-chain or branched alkyl having 1 to 4 carbon atoms or cyano and |
| $R^6$ | represents hydrogen or straight-chain or branched alkyl having 1 to 4 carbon atoms or |
| $R^5$ and $R^6$ | together represent divalent alkanediyl having 4 to 9 carbon atoms, |
| A | represents one of the radicals $-CH_2-CH_2-$; $-CH(CH_3)-CH_2-$ or $-CH_2-O-$ and |
| X | represents oxygen or sulphur, but with the exception of the compound 5-dimethylamino-4-methyl-3-(4-phenylbut-2-ylaminocarbonyl)-1,2,4-triazole. |

3. Process for the preparation of substituted triazoles of the general formula (I) according to Claim 1, in which

| | |
|---|---|
| $R^1$ | represents methyl, |
| $R^2$ | represents methyl or ethyl, |
| $R^3$ | represents methyl or ethyl, |
| $R^4$ | represents phenyl which is optionally monosubstituted to trisubstituted by identical or different substituents, suitable substituents being: fluorine, chlorine, bromine, cyano, nitro, methyl, ethyl, n- or i-propyl, n-, i-, s- or t-butyl, methoxy, ethoxy, n- or i-propoxy, n-, i-, s- or t-butoxy, methylthio, ethylthio, difluoromethyl, trifluoromethyl, difluoromethoxy, trifluoromethoxy, difluoromethylthio, trifluoromethylthio, dimethylamino, diethylamino, N-acetamido, dioxymethylene, difluorodioxymethylene, dioxyethylene, trifluorodioxyethylene, tetrafluorodioxyethylene, methoxycarbonyl, ethoxycarbonyl, propoxycarbonyl, methoximinomethyl, methoximinoethyl, ethoximinomethyl, ethoximinoethyl, or phenyl, phenoxy, α-naphthyl or β-naphthyl, each of which is optionally monosubstituted to trisubstituted by identical or different substituents from the series comprising fluorine, chlorine, bromine, methyl, ethyl, methoxy and/or ethoxy, |
| $R^5$ | represents hydrogen, methyl, ethyl or cyano, |
| $R^6$ | represents methyl, ethyl, n- or i-propyl, |
| A | represents one of the radicals $-CH_2-CH_2-$; $-CH(CH_3)-CH_2-$ or $-CH_2-O-$ and |
| X | represents oxygen, |

but with the exception of the compound 5-dimethylamino-4-methyl-3-(4-phenylbut-2-ylaminocarbonyl)-1,2,4-triazole.

4. Herbicidal agents, characterized in that they contain at least one substituted triazole of the formula (I) according to Claims 1 to 3.

5. Process for combating undesired plants, characterized in that substituted triazoles of the formula (I) according to Claims 1 to 3 are allowed to act on undesired plants and/or their environment.

6. Use of substituted triazoles of the formula (I) according to Claims 1 to 3 for combating undesired plants.

7. Process for the preparation of herbicidal agents, characterized in that substituted triazoles of the formula (I) according to Claims 1 to 3 are mixed with extenders and/or surface-active substances.

**Revendications**

**Revendications pour les Etats contractants suivants : BE, CH, DE, DK, FR, GB, IT, LI, NL**

1. Triazoles substitués de formule générale (I)

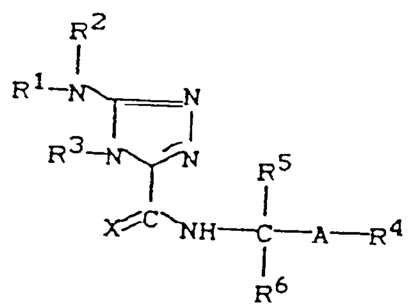

(I)

dans lesquels

| | |
|---|---|
| $R^1$ | est un groupe alkyle à chaîne droite ou ramifiée ayant 1 à 6 atomes de carbone, |
| $R^2$ | est un groupe alkyle à chaîne droite ou ramifiée ayant 1 à 6 atomes de carbone, |
| $R^3$ | est un groupe alkyle à chaîne droite ou ramifiée ayant 1 à 6 atomes de carbone, |
| $R^4$ | est un groupe aryle de 6 ou 10 atomes de carbone portant le cas échéant un ou plusieurs substituants identiques ou différents, en considérant comme substituants : un halogène, un groupe cyano, nitro, un groupe alkyle, alkoxy ou alkylthio à chaîne droite ou ramifiée ayant chacun 1 à 4 atomes de carbone, un groupe halogénalkyle, halogénalkoxy, halogénalkylthio à chaîne droite ou ramifiée ayant chacun 1 à 4 atomes de carbone et 1 à 9 atomes d'halogènes identiques ou différents, un groupe alkoxycarbonyle ou alkoximinoalkyle à chaîne droite ou ramifiée ayant chacun 1 à 4 atomes de carbone dans les parties alkyle individuelles, un groupe dialkylamino ayant 1 à 4 atomes de carbone dans les parties alkyle individuelles à chaîne droite ou ramifiée, un groupe N-alcanoylamino ayant 1 à 5 atomes de carbone dans la partie alcanoyle à chaîne droite ou ramifiée, un groupe dioxyalkylène divalent ayant 1 à 3 atomes de carbone, portant le cas échéant un ou plusieurs substituants halogéno identiques ou différents, ainsi que le cas échéant un groupe phényle, phénoxy, α-naphtyle ou β-naphtyle portant le cas échéant un ou plusieurs substituants, identiques ou différents, halogéno et/ou alkyle à chaîne droite ou ramifiée ayant 1 à 4 atomes de carbone, et $R^4$ représente en outre un groupe indanyle, |
| $R^5$ | est de l'hydrogène, un groupe alkyle à chaîne droite ou ramifiée ayant 1 à 6 atomes de carbone ou un groupe cyano, |
| $R^6$ | est un atome d'hydrogène ou un groupe alkyle à chaîne droite ou ramifiée ayant 1 à 6 atomes de carbone, ou bien |
| $R^5$ et $R^6$ | forment conjointement un groupe alcanediyle divalent ayant 2 à 9 atomes de carbone, |
| A | est un reste de formule $-CH_2CH_2-$ ; $-CH(CH_3)-CH_2-$ ; $-CH_2O-$ et |
| X | représente de l'oxygène ou du soufre |

à l'exclusion toutefois du composé 5-diméthylamino-4-méthyl-3-(4-phénylbut-2-ylaminocarbonyl)-1,2,4-triazole.

2. Triazoles substitués de formule générale (I) suivant la revendication, dans lesquels

| | |
|---|---|
| $R^1$ | est un groupe alkyle à chaîne droite ou ramifiée ayant 1 à 4 atomes de carbone, |
| $R^2$ | est un groupe alkyle à chaîne droite ou ramifiée ayant 1 à 4 atomes de carbone, |
| $R^3$ | est un groupe alkyle à chaîne droite ou ramifiée ayant 1 à 4 atomes de carbone, |
| $R^4$ | est un groupe phényle, α-naphtyle, β-naphtyle ou indanyle portant chacun le cas échéant 1 à 3 substituants identiques ou différents, en considérant dans chaque cas comme substituants : |

le fluor, le chlore, le brome, un groupe cyano, nitro, méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylthio, éthylthio, difluorométhyle, trifluorométhyle, difluorométhoxy, trifluorométhoxy, difluorométhylthio, trifluorométhylthio, diméthylamino, diéthylamino, N-acétamido, dioxyméthylène, difluorodioxyméthylène, dioxyéthylène, trifluorodioxyéthylène, tétrafluorodioxyéthylène, méthoxycarbonyle, éthoxycarbonyle, propoxycarbonyle, méthoximinométhyle, méthoximinoéthyle, éthoximinométhyle, éthoximinoéthyle ou un groupe phényle, phénoxy, (α-naphtyle ou β-naphtyle portant chacun le cas échéant 1 à 3 substituants, identiques ou différents, fluoro, chloro, bromo, méthyle, éthyle, méthoxy et/ou éthoxy,

$R^5$ représente de l'hydrogène, un groupe alkyle à chaîne droite ou ramifiée ayant 1 à 4 atomes de carbone ou un groupe cyano, et

$R^6$ est de l'hydrogène ou un groupe alkyle à chaîne droite ou ramifiée ayant 1 à 4 atomes de carbone, ou bien

$R^5$ et $R^6$ forment conjointement un groupe alcanediyle divalent ayant 4 à 9 atomes de carbone,

A représente l'un des reste $-CH_2-CH_2-$ ; $-CH(CH_3)-CH_2$ ; $-CH_2-O-$ ; et

X est de l'oxygène ou du soufre,

à l'exclusion toutefois du composé 5-diméthylamino-4-méthyl-3-(4-phénylbut-2-ylaminocarbonyl)-1,2,4-triazole.

3. Triazoles substitués de formule générale (I) suivant la revendication 1, dans lesquels

$R^1$ est un groupe méthyle,

$R^2$ est un groupe méthyle ou éthyle,

$R^3$ est un groupe méthyle ou éthyle,

$R^4$ est un groupe phényle portant le cas échéant 1 à 3 substituants identiques ou différents, en considérant comme substituants :
le fluor, le chlore, le brome, un groupe cyano, nitro, méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylthio, éthylthio, difluorométhyle, trifluorométhyle, difluorométhoxy, trifluorométhoxy, difluorométhylthio, trifluorométhylthio, diméthylamino, diéthylamino, N-acétamido, dioxyméthylène, difluorodioxyméthylène, dioxyéthylène, trifluorodioxyéthylène, tétrafluorodioxyéthylène, méthoxycarbonyle, éthoxycarbonyle, propoxycarbonyle, méthoximinométhyle, méthoximinoéthyle, éthoximinométhyle, éthoximinoéthyle ou un groupe phényle, phénoxy, α-naphtyle ou β-naphtyle portant le cas échéant 1 à 3 substituants, identiques ou différents, fluoro, chloro, bromo, méthyle, éthyle, méthoxy et/ou éthoxy,

$R^5$ est un atome d'hydrogène, un groupe méthyle, éthyle ou cyano,

$R^6$ est un groupe méthyle, éthyle, n-propyle ou isopropyle,

A représente l'un des restes $-CH_2-CH_2-$ ; $-CH(CH_3)-CH_2$ ; $-CH_2-O-$ ; et

X est de l'oxygène,

à l'exception toutefois du composé 5-diméthylamino-4-méthyl-3-(4-phénylbut-2-ylaminocarbonyl)-1,2,4-triazole.

4. Procédé de production de triazoles substitués de formule générale (I) suivant la revendication 1

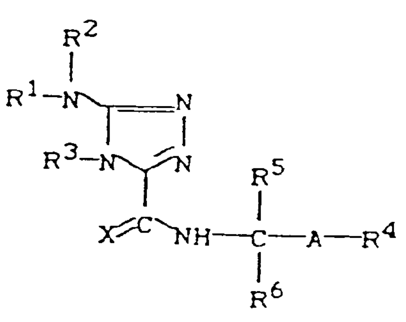

(I)

dans lesquels

| | |
|---|---|
| $R^1$ | est un groupe alkyle à chaîne droite ou ramifiée ayant 1 à 6 atomes de carbone, |
| $R^2$ | est un groupe alkyle à chaîne droite ou ramifiée ayant 1 à 6 atomes de carbone, |
| $R^3$ | est un groupe alkyle à chaîne droite ou ramifiée ayant 1 à 6 atomes de carbone, |
| $R^4$ | est un groupe aryle de 6 ou 10 atomes de carbone portant le cas échéant un ou plusieurs substituants identiques ou différents, en considérant comme substituants : un halogène, un groupe cyano, nitro, un groupe alkyle, alkoxy ou alkylthio à chaîne droite ou ramifiée ayant chacun 1 à 4 atomes de carbone, un groupe halogénalkyle, halogénalkoxy, halogénalkylthio à chaîne droite ou ramifiée ayant chacun 1 à 4 atomes de carbone et 1 à 9 atomes d'halogènes identiques ou différents, un groupe alkoxycarbonyle ou alkoximinoalkyle à chaîne droite ou ramifiée ayant chacun 1 à 4 atomes de carbone dans les parties alkyle individuelles, un groupe dialkylamino ayant 1 à 4 atomes de carbone dans les parties alkyle individuelles à chaîne droite ou ramifiée, un groupe N-alcanoylamino ayant 1 à 5 atomes de carbone dans la partie alcanoyle à chaîne droite ou ramifiée, un groupe dioxyalkylène divalent ayant 1 à 3 atomes de carbone, portant le cas échéant un ou plusieurs substituants halogéno identiques ou différents, ainsi que le cas échéant un groupe phényle, phénoxy, α-naphtyle ou β-naphtyle portant le cas échéant un ou plusieurs substituants, identiques ou différents, halogéno et/ou alkyle à chaîne droite ou ramifiée ayant 1 à 4 atomes de carbone, et $R^4$ représente en outre un groupe indanyle, |
| $R^5$ | est de l'hydrogène, un groupe alkyle à chaîne droite ou ramifiée ayant 1 à 6 atomes de carbone ou un groupe cyano, |
| $R^6$ | est un atome d'hydrogène ou un groupe alkyle à chaîne droite ou ramifiée ayant 1 à 6 atomes de carbone, ou bien |
| $R^5$ et $R^6$ | forment conjointement un groupe alcanediyle divalent ayant 2 à 9 atomes de carbone, |
| A | est un reste de formule $-CH_2CH_2-$ ; $-CH(CH_3)-CH_2-$ ; $-CH_2-O-$ et |
| X | représente de l'oxygène ou du soufre, |

à l'exclusion toutefois du composé 5-diméthylamino-4-méthyl-3-(4-phénylbut-2-ylaminocarbonyl)-1,2,4-triazole,

caractérisé en ce qu'on fait réagir,

a) des aminoguanidines de formule (II),

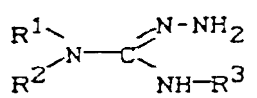

(II)

dans laquelle

$R^1$, $R^2$ et $R^3$ ont la définition indiquée ci-dessus,

ou leurs sels d'addition d'acides avec des (thio)oxalesteramides de formule (III),

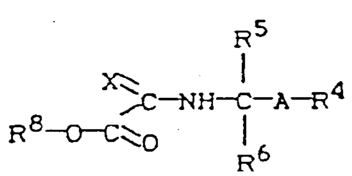

(III)

dans laquelle

$R^4$, $R^5$, $R^6$, X et A ont la definition indiquée ci-dessus, et

$R^8$ est un groupe alkyle à chaîne droite ou ramifiée ayant 1 à 4 atomes de carbone,

le cas échéant en présence d'un diluant et en la présence éventuelle d'une substance auxiliaire de réaction,

ou bien,

b) on fait réagir des esters d'acides triazolyl(thio)carboxyliques substitués de formule (IV),

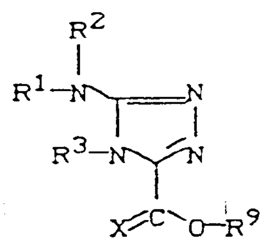

(IV)

dans laquelle
$R^1$, $R^2$, $R^3$ et X ont la définition indiquée ci-dessus et
$R^9$ est un groupe alkyle à chaîne droite ou ramifiée ayant 1 à 4 atomes de carbone,
avec des amines de formule (V),

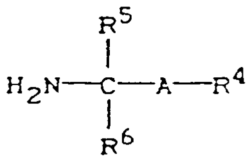

(V)

dans laquelle
$R^4$, $R^5$, $R^6$ et A ont la définition indiquée ci-dessus, le cas échéant en présence d'un diluant.

5. Compositions herbicides, caractérisées par une teneur en au moins un triazole substitué de formule (I) suivant les revendications 1 à 4.

6. Procédé pour combattre des plantes non désirées, caractérisé en ce qu'on fait agir sur les plantes non désirées et/ou sur leur milieu, des triazoles substitués de formule (I) suivant les revendications 1 à 4.

7. Utilisation de triazoles substitués de formule (I) suivant les revendications 1 à 4 pour combattre des plantes non désirées.

8. Procédé de préparation de compositions herbicides, caractérisé en ce qu'on mélange des triazoles substitués de formule (I) suivant les revendications 1 à 4 avec des diluants et/ou des substances tensioactives.

**Revendications pour l'Etat contractant suivant : ES**

1. Procédé de production de triazoles substitués de formule générale (I)

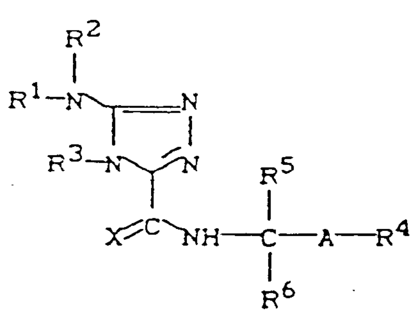

(I)

dans lesquels
$R^1$ est un groupe alkyle à chaîne droite ou ramifiée ayant 1 à 6 atomes de carbone,
$R^2$ est un groupe alkyle à chaîne droite ou ramifiée ayant 1 à 6 atomes de carbone,

| | |
|---|---|
| $R^3$ | est un groupe alkyle à chaîne droite ou ramifiée ayant 1 à 6 atomes de carbone, |
| $R^4$ | est un groupe aryle de 6 ou 10 atomes de carbone portant le cas échéant un ou plusieurs substituants identiques ou différents, en considérant comme substituants : un halogène, un groupe cyano, nitro, un groupe alkyle, alkoxy ou alkylthio à chaîne droite ou ramifiée ayant chacun 1 à 4 atomes de carbone, un groupe halogénalkyle, halogénalkoxy, halogénalkylthio à chaîne droite ou ramifiée ayant chacun 1 à 4 atomes de carbone et 1 à 9 atomes d'halogènes identiques ou différents, un groupe alkoxycarbonyle ou alkoximinoalkyle à chaîne droite ou ramifiée ayant chacun 1 à 4 atomes de carbone dans les parties alkyle individuelles, un groupe dialkylamino ayant 1 à 4 atomes de carbone dans les parties alkyle individuelles à chaîne droite ou ramifiée, un groupe N-alcanoylamino ayant 1 à 5 atomes de carbone dans la partie alcanoyle à chaîne droite ou ramifiée, un groupe dioxyalkylène divalent ayant 1 à 3 atomes de carbone, portant le cas échéant un ou plusieurs substituants halogéno identiques ou différents, ainsi que le cas échéant un groupe phényle, phénoxy, α-naphtyle ou β-naphtyle portant le cas échéant un ou plusieurs substituants, identiques ou différents, halogéno et/ou alkyle à chaîne droite ou ramifiée ayant 1 à 4 atomes de carbone, et $R^4$ représente en outre un groupe indanyle, |
| $R^5$ | est de l'hydrogène, un groupe alkyle à chaîne droite ou ramifiée ayant 1 à 6 atomes de carbone ou un groupe cyano, |
| $R^6$ | est un atome d'hydrogène ou un groupe alkyle à chaîne droite ou ramifiée ayant 1 à 6 atomes de carbone, ou bien |
| $R^5$ et $R^6$ | forment conjointement un groupe alcanediyle divalent ayant 2 à 9 atomes de carbone, |
| A | est un reste de formule $-CH_2CH_2-$ ; $-CH(CH_3)-CH_2-$ ; $-CH_2O-$ et |
| X | représente de l'oxygène ou du soufre |

à l'exclusion toutefois du composé 5-diméthylamino-4-méthyl-3-(4-phénylbut-2-ylaminocarbonyl)-1,2,4-triazole,

caractérisé en ce qu'on fait réagir,

a) des aminoguanidines de formule (II),

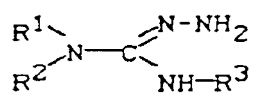

(II)

dans laquelle

$R^1$, $R^2$ et $R^3$ ont la définition indiquée ci-dessus,

ou leurs sels d'addition d'acides avec des (thio)oxalesteramides de formule (III),

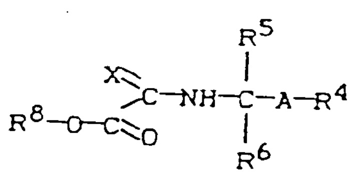

(III)

dans laquelle

$R^4$, $R^5$, $R^6$, X et A ont la définition indiquée ci-dessus, et

$R^8$ est un groupe alkyle à chaîne droite ou ramifiée ayant 1 à 4 atomes de carbone,

le cas échéant en présence d'un diluant et en la presence éventuelle d'une substance auxiliaire de réaction,

ou bien,

b) on fait réagir des esters d'acides triazolyl(thio)carboxyliques substitués de formule (IV),

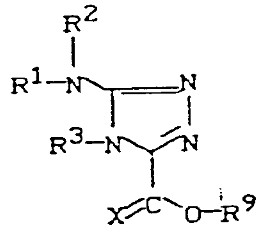

(IV)

dans laquelle

$R^1$, $R^2$, $R^3$ et X ont la définition indiquée ci-dessus et

$R^9$ est un groupe alkyle à chaîne droite ou ramifiée ayant 1 à 4 atomes de carbone,

avec des amines de formule (V),

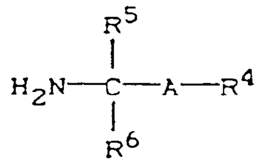

(V)

dans laquelle

$R^4$, $R^5$, $R^6$ et A ont la définition indiquée ci-dessus, le cas échéant en présence d'un diluant.

2. Procédé de production de triazoles substitués de formule générale (I) suivant la revendication 1, dans lesquels
   - $R^1$ est un groupe alkyle à chaîne droite ou ramifiée ayant 1 à 4 atomes de carbone,
   - $R^2$ est un groupe alkyle à chaîne droite ou ramifiée ayant 1 à 4 atomes de carbone,
   - $R^3$ est un groupe alkyle à chaîne droite ou ramifiée ayant 1 à 4 atomes de carbone,
   - $R^4$ est un groupe phényle, α-naphtyle, β-naphtyle ou indanyle portant chacun le cas échéant 1 à 3 substituants identiques ou différents, en considérant dans chaque cas comme substituants :
     le fluor, le chlore, le brome, un groupe cyano, nitro, méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylthio, éthylthio, difluorométhyle, trifluorométhyle, difluorométhoxy, trifluorométhoxy, difluorométhylthio, trifluorométhylthio, diméthylamino, diéthylamino, N-acétamido, dioxyméthylène, difluorodioxyméthylène, dioxyéthylène, trifluorodioxyéthylène, tétrafluorodioxyéthylène , méthoxycarbonyle, éthoxycarbonyle, propoxycarbonyle, méthoximinométhyle, méthoximinoéthyle, éthoximinométhyle, éthoximinoéthyle ou un groupe phényle, phénoxy, α-naphtyle ou β-naphtyle portant chacun le cas échéant 1 à 3 substituants, identiques ou différents, fluoro, chloro, bromo, méthyle, éthyle, méthoxy et/ou éthoxy,
   - $R^5$ représente de l'hydrogène, un groupe alkyle à chaîne droite ou ramifiée ayant 1 à 4 atomes de carbone ou un groupe cyano,
   - $R^6$ est de l'hydrogène ou un groupe alkyle à chaîne droite ou ramifiée ayant 1 à 4 atomes de carbone, ou bien
   - $R^5$ et $R^6$ forment conjointement un groupe alcanediyle divalent ayant 4 à 9 atomes de carbone,
   - A représente l'un des reste $-CH_2-CH_2-$ ; $-CH(CH_3)-CH_2$ ; $CH_2-O-$ ; et
   - X est de l'oxygène ou du soufre,

   à l'exclusion toutefois du composé 5-diméthylamino-4-méthyl-3-(4-phénylbut-2-ylaminocarbonyl)-1,2,4-triazole.

3. Procédé de production de triazoles substitués de formule générale (I) suivant la revendication 1, dans lesquels :

$R^1$ est un groupe méthyle,
$R^2$ est un groupe méthyle ou éthyle,
$R^3$ est un groupe méthyle ou éthyle,
$R^4$ est un groupe phényle portant le cas échéant 1 à 3 substituants identiques ou différents, en considerant comme substituants :
le fluor, le chlore, le brome, un groupe cyano, nitro, méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylthio, éthylthio, difluorométhyle, trifluorométhyle, difluorométhoxy, trifluorométhoxy, difluorométhylthio, trifluorométhylthio, diméthylamino, diéthylamino, N-acétamido, dioxyméthylène, difluorodioxyméthylène, dioxyéthylène, trifluorodioxyéthylène, tétrafluorodioxyéthylène, méthoxycarbonyle, éthoxycarbonyle, propoxycarbonyle, méthoximinométhyle, méthoximinoé- thyle, éthoximinométhyle, éthoximinoéthyle ou un groupe phényle, phénoxy, $\alpha$-naphtyle ou $\beta$-naphtyle portant le cas échéant 1 à 3 substituants, identiques ou différents, fluoro, chloro, bromo, méthyle, éthyle, méthoxy et/ou éthoxy,
$R^5$ est un atome d'hydrogène, un groupe méthyle, éthyle ou cyano,
$R^6$ est un groupe méthyle, éthyle, n-propyle ou isopropyle,
A représente l'un des restes $-CH_2-CH_2-$ ; $-CH(CH_3)-CH_2$ ; $-CH_2-O-$ ; et
X est de l'oxygène, à l'exception toutefois du composé 5-diméthylamino-4-méthyl-3-(4-phénylbut-2-ylaminocarbonyl)-1,2,4,-triazole.

**4.** Compositions herbicides, caractérisées par une teneur en au moins un triazole substitué de formule (I) suivant les revendications 1 à 3.

**5.** Procédé pour combattre des plantes non désirées, caractérisé en ce qu'on fait agir sur les plantes non désirées et/ou sur leur milieu des triazoles substitués de formule (I) suivant les revendications 1 à 3.

**6.** Utilisation de triazoles substitués de formule (I) suivant les revendications 1 à 3 pour combattre des plantes non désirées.

**7.** Procédé de préparation de compositions herbicides, caractérisé en ce qu'on mélange des triazoles substitués de formule (I) suivant les revendications 1 à 3 avec des diluants et/ou des substances tensioactives.